(19) Europäisches Patentamt
European Patent Office
Office européen des brevets

(11) EP 3 150 720 B1

(12) **EUROPEAN PATENT SPECIFICATION**

(45) Date of publication and mention of the grant of the patent:
**12.12.2018 Bulletin 2018/50**

(51) Int Cl.:
***C12Q 1/68*** *(2018.01)*

(21) Application number: **16184484.0**

(22) Date of filing: **30.07.2012**

(54) **METHOD FOR PREDICTING THE RESPONSE TO CHEMOTHERAPY IN A PATIENT SUFFERING FROM OR AT RISK OF DEVELOPING RECURRENT BREAST CANCER**

VERFAHREN ZUR VORHERSAGE DER REAKTION AUF EINE CHEMOTHERAPIE BEI EINEM PATIENT MIT BRUSTKREBSREZIDIV ODER RISIKO FÜR BRUSTKREBSREZIDIV

PROCÉDÉ DE PRÉDICTION DE LA RÉPONSE À UNE CHIMIOTHÉRAPIE CHEZ UNE PATIENTE SOUFFRANT OU RISQUANT DE DÉVELOPPER UN CANCER DU SEIN RÉCURRENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2011 EP 11175852**

(43) Date of publication of application:
**05.04.2017 Bulletin 2017/14**

(62) Document number(s) of the earlier application(s) in accordance with Art. 76 EPC:
**12740974.6 / 2 737 081**

(73) Proprietor: **Myriad International GmbH**
**50829 Cologne (DE)**

(72) Inventors:
- **GEHRMANN, Mathias**
  **50829 Cologne (DE)**
- **WEBER, Karsten**
  **50829 Cologne (DE)**
- **KRONENWETT, Ralf**
  **50829 Cologne (DE)**
- **PETRY, Christoph**
  **50829 Cologne (DE)**
- **BRASE, Jan**
  **50829 Cologne (DE)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2010/076322** **WO-A1-2011/120984**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to methods for predicting the response of a tumor to chemotherapy. More specific, the present invention relates to the prediction of the response to chemotherapeutic agents, in particular but not limited to a neoadjuvant setting based on the measurements of gene expression levels in tumor samples of breast cancer patients.

### Background of the Invention

Breast cancer is the most common tumor type and one of the leading causes of cancer-related death in women (Jemal et al., CA Cancer J Clin., 2011). It is estimated that every tenth woman will develop breast cancer during her lifetime. Although the incidence has increased over the years, the mortality has constantly decreased due to the advances in early detection and the development of novel effective treatment strategies.

Breast cancer patients are frequently treated with radiotherapy, hormone therapy or cytotoxic chemotherapy after surgery (adjuvant treatment) to control for residual tumor cells and reduce the risk of recurrence. Chemotherapy includes the combined use of several cytotoxic agents, whereas anthracycline and taxane-based treatment strategies have been shown to be superior compared to other standard combination therapies (Misset et al.,J Clin Oncol., 1996, Henderson et al., J Clin Oncol., 2003).

Systemic chemotherapy is commonly applied to reduce the likelihood of recurrence in HER2/neu-positive and in tumors lacking expression of the estrogen receptor and HER2/neu receptor (triple negative, basal). The most challenging treatment decision concerns luminal (estrogen receptor positive and HER2/neu-negative) tumors for which classical clinical factors like grading, tumor size or lymph node involvement do not provide a clear answer to the question whether to use chemotherapy or not.

To reduce the number of patients suffering from serious side effects without a clear benefit of systemic therapy, there is a great need for novel molecular biomarkers to predict the sensitivity to chemotherapy and thus allow a more tailored treatment strategy.

Chemotherapy can also be applied in the neoadjuvant (preoperative) setting in which breast cancer patients receive systemic therapy before the remaining tumor cells are removed by surgery. Neoadjuvant chemotherapy of early breast cancer leads to high clinical response rates of 70-90%. However, in the majority of clinical responders, the pathological assessment of the tumor residue reveals the presence of residual tumor cell foci. A complete eradication of cancer cells in the breast and lymph nodes after neoadjuvant treatment is called pathological complete response (pCR) and observed in only 10-25% of all patients. The pCR is an appropriate surrogate marker for disease-free survival and a strong indicator of benefit from chemotherapy.

The preoperative treatment strategy provides the opportunity to directly assess the response of a particular tumor to the applied therapy: the reduction of the tumor mass in response to therapy can be directly monitored. For patients with a low probability of response, other therapeutic approaches should be considered. Biomarkers can be analyzed from pretherapeutic core biopsies to identify the most valuable predictive markers. A common approach is to isolate RNA from core biopsies for the gene expression analysis before neoadjuvant therapy. Afterwards the therapeutic success can be directly evaluated by the tumor reduction and correlated with the gene expression data.

Predictive multigene assays like the DLDA30 (Hess et al., J Clin Oncol., 2006) have been shown to provide information beyond clinical parameters like tumor grading and hormone receptor status in breast cancer patients treated with neoadjuvant therapy. However, the predictive multigene test DLDA30 was established without considering the estrogen receptor status. Therefore the test might reflect phenotypic differences between complete responder and nonresponder, responders being predominantly ER-negative and HER2/neu positive (Tabchy et al., Clin Can Res, 2010).

Additionally, established multigene tests for prognosis were analyzed in the neoadjuvant setting to assess whether the prognostic assays can also predict chemosensitivity. One example is the Genomic Grade Index (GGI), a multigene test to define histologic grade based on gene expression profiles (Sotiriou et al, JNCI, 2006). It was demonstrated by Liedtke and colleagues that a high GGI is associated with increased chemosensitivity in breast cancer patients treated with neoadjuvant therapy (Liedtke, J Clin Oncol, 2009).

Although gene signatures have been shown to predict the therapy response, large-scale validation studies including clinical follow-up data are missing and so far none of them is commonly used to guide treatment decisions in clinical routine as yet.

WO2010/076322 A1 discloses a method for predicting a response to and/or benefit from chemotherapy in a patient suffering from cancer comprising the steps of (i) classifying a tumor into at least two classes, (ii) determining in a tumor sample the expression of at least one marker gene indicative of a response to chemotherapy for a tumor in each respective class, (iii) depending on said gene expression, predicting said response and/or benefit ; wherein said at least one marker gene comprises a gene selected from the group consisting of TMSL8, ABCC1, EGFR, MVP, ACOX2, HER2/NEU, MYH11, TOB1, AKR1C1, ERBB4, NFKB1A, TOP2A, AKR1C3, ESR1, OLFM1, TOP2B, ALCAM, FRAP1, PGR, TP53, BCL2, GADD45A, PRKAB1, TUBA1A, C16orf45, HIF1A, PTPRC, TUBB, CA12, IGKC, RACGAP1, UBE2C, CD14, 1KBKB, S100A7, VEGFA, CD247, KRT5, SEPT8, YBX1, CD3D, MAPK3, SLC2A1, CDKN1A, MAPT, SLC7A8, CHPT1, MLPH, SPON1, CXCL13, MMP1, STAT1, CXCL9, MMP7, STC2, DCN, MUC1, STMN1 and combinations thereof.

Maia Chanrion et al. report in Clin Cancer Res 2008; 14(6) March 15, 2008, p. 1744-1752 about a gene expression signature that can predict the recurrence of tamoxifen-treated primary breast cancer. The disclosed study identifies a molecular signature specifying a subgroup of patients who do not gain benefits from tamoxifen treatment. These patients may therefore be eligible for alternative endocrine therapies and/or chemotherapy.

WO 2009/158143A1 discloses methods for classifying and for evaluating the prognosis of a subject having breast cancer are provided. The methods include prediction of breast cancer subtype using a supervised algorithm trained to stratify subjects on the basis of breast cancer intrinsic subtype. The prediction model is based on the gene expression profile of the intrinsic genes listed in Table 1. This prediction model can be used to accurately predict the intrinsic subtype of a subject diagnosed with or suspected of having breast cancer. Further provided are compositions and methods for predicting outcome or response to therapy of a subject diagnosed with or suspected of having breast cancer. These methods are useful for guiding or determining treatment options for a subject afflicted with breast cancer. Methods of the invention further include means for evaluating gene expression profiles, including microarrays and quantitative polymerase chain reaction assays, as well as kits comprising reagents for practicing the methods of the invention

WO 2006/119593 discloses methods and systems for prognosis determination in tumor samples, by measuring gene expression in a tumor sample and applying a gene-expression grade index (GGI) or a relapse score (RS) to yield a numerical risk score

Karen J Taylor et al. report in Breast Cancer Research 2010, 12:R39 about dynamic changes in gene expression *in vivo to* predict prognosis of tamoxifen-treated patients with breast cancer.

WO 2008/006517A2 discloses methods and kits for the prediction of a likely outcome of chemotherapy in a cancer patient. More specifically, the invention relates to the prediction of tumor response to chemotherapy based on measurements of expression levels of a small set of marker genes. The set of marker genes is useful for the identification of breast cancer subtypes responsive to taxane based chemotherapy, such as e.g. a taxane-anthracycline-cyclophosphamide-based (e.g. Taxotere (docetaxel)- Adriamycin (doxorubicin)-cyclophosphamide, i.e. (TAC)-based) chemotherapy.

WO 2009/114836 A1 discloses gene sets which are useful in assessing prognosis and/or predicting the response of cancer, e.g. colorectal cancer to chemotherapy, are disclosed. Also disclosed is a clinically validated cancer test, e.g. colorectal test, for assessment of prognosis and/or prediction of patient response to chemotherapy, using expression analysis. The use of archived paraffin embedded biopsy material for assay of all markers in the relevant gene sets is accomodated for, and therefore is compatible with the most widely available type of biopsy material.

WO 2011/120984A1 discloses methods, kits and systems for the prognosis of the disease outcome of breast cancer, said method comprising : (a) determining in a tumor sample from said patient the RNA expression levels of at least 2 of the following 9 genes: UBE2C, BIRC5, RACGAP1, DHCR7, STC2, AZGP1, RBBP8, IL6ST, and MGP (b) mathematically combining expression level values for the genes of the said set which values were determined in the tumor sample to yield a combined score, wherein said combined score is indicative of a prognosis of said patient; and kits and systems for performing said method.

**Definitions**

Unless defined otherwise, technical and scientific terms used herein have the same meaning as commonly understood by one of ordinary skill in the art to which this invention belongs.

"Predicting the response to chemotherapy", within the meaning of the invention, shall be understood to be the act of determining a likely outcome of cytotoxic chemotherapy in a patient affected by cancer. The prediction of a response is preferably made with reference to probability values for reaching a desired or non-desired outcome of the chemotherapy. The predictive methods of the present invention can be used clinically to make treatment decisions by choosing the most appropriate treatment modalities for any particular patient.

The "response of a tumor to chemotherapy", within the meaning of the invention, relates to any response of the tumor to cytotoxic chemotherapy, preferably to a change in tumor mass and/or volume after initiation of neoadjuvant chemotherapy and/or prolongation of time to distant metastasis or time to death following neoadjuvant or adjuvant chemotherapy. Tumor response may be assessed in a neoadjuvant situation where the size of a tumor after systemic intervention can be compared to the initial size and dimensions as measured by CT, PET, mammogram, ultrasound or palpation, usually recorded as "clinical response" of a patient. Response may also be assessed by caliper measurement or pathological examination of the tumor after biopsy or surgical resection. Response may be recorded in a quantitative fashion like percentage change in tumor volume or in a qualitative fashion like "no change" (NC), "partial remission" (PR), "complete remission" (CR) or other qualitative criteria. Assessment of tumor response may be done early after the onset of neoadjuvant therapy e.g. af-ter a few hours, days, weeks or preferably after a few months. A typical endpoint for response assessment is upon termination of neoadjuvant chemotherapy or upon surgical removal of residual tumor cells and/or the tumor bed. This is typically three month after initiation of neoadjuvanttherapy. Response may also be assessed by comparing time to distant metastasis or death of a patient following neoadjuvant or adjuvant chemotherapy with time to distant metastasis or death of a patient not treated with chemotherapy.

The term "tumor" as used herein, refers to all neoplastic cell growth and proliferation, whether malignant or benign, and all pre-cancerous and cancerous cells and tissues.

The term "cancer" refer to or describe the physiological condition in mammals that is typically characterized by unregulated cell growth. The term "cancer" as used herein includes carcinomas, (e.g., carcinoma in situ, invasive carcinoma, metastatic carcinoma) and pre-malignant con-ditions, neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer and primary carcinomas are included.

The term "cytotoxic chemotherapy" refers to various treatment modalities affecting cell proliferation and/or survival. The treatment may include administration of alkylating agents, antimetabolites, anthracyclines, plant alkaloids, topoisomerase inhibitors, and other antitumor agents, including monoclonal antibodies and kinase inhibitors. In particular, the cytotoxic treatment may relate to a taxane treatment. Taxanes are plant alkaloids which block cell division by preventing microtubule function. The prototype taxane is the natural product paclitaxel, originally known as Taxol and first derived from the bark of the Pacific Yew tree. Docetaxel is a semi-synthetic analogue of paclitaxel. Taxanes enhance stability of microtubules, preventing the separation of chromosomes during anaphase.

The term "therapy" refers to a timely sequential or simultaneous administration of anti-tumor, and/or anti vascular, and/or anti stroma, and/or immune stimulating or suppressive, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may vary in the dose of each of the single agents, timeframe of application and frequency of administration within a defined therapy window. Currently various combinations of various drugs and/or physical methods, and various schedules are under investigation. A "taxane/anthracycline-containing chemotherapy" is a therapy modality comprising the administration of taxane and/or anthracycline and therapeutically effective derivates thereof.

The term "neoadjuvant chemotherapy" relates to a preoperative therapy regimen consisting of a panel of hormonal, chemotherapeutic and/or antibody agents, which is aimed to shrink the primary tumor, thereby rendering local therapy (surgery or radiotherapy) less destructive or more effective, enabling breast conserving surgery and evaluation of responsiveness of tumor sensitivity towards specific agents in vivo.

The term "lymph node involvement" means a patient having previously been diagnosed with lymph node metastasis. It shall encompass both draining lymph node, near lymph node, and distant lymph node metastasis. This previous diagnosis itself shall not form part of the inventive method. Rather it is a precondition for selecting patients whose samples may be used for one embodiment of the present invention. This previous diagnosis may have been arrived at by any suitable method known in the art, including, but not limited to lymph node removal and pathological analysis, biopsy analysis, in-vitro analysis of biomarkers indicative for metastasis, imaging methods (e.g. computed tomography, X-ray, magnetic resonance imaging, ultrasound), and intraoperative findings.

The term "pathological complete response" (pCR), as used herein, relates to a complete disappearance or absence of invasive tumor cells in the breast and/or lymph nodes as assessed by a histopathological examination of the surgical specimen following neoadjuvant chemotherapy.

The term "marker" or "biomarker" refers to a biological molecule, e.g., a nucleic acid, peptide, protein, hormone, etc., whose presence or concentration can be detected and correlated with a known condition, such as a disease state.

The term "predictive marker" relates to a marker which can be used to predict the clinical response of a patient towards a given treatment.

The term "prognosis", as used herein, relates to an individual assessment of the malignancy of a tumor, or to the expected response if there is no drug therapy. In contrast thereto, the term "prediction" relates to an individual assessment of the malignancy of a tumor, or to the expected response if the therapy contains a drug in comparison to the malignancy or response without this drug.

The term "immunohistochemistry" or IHC refers to the process of localizing proteins in cells of a tissue section exploiting the principle of antibodies binding specifically to antigens in biological tissues. Immunohistochemical staining is widely used in the diagnosis and treatment of cancer. Specific molecular markers are characteristic of particular cancer types. IHC is also widely used in basic research to understand the distribution and localization of biomarkers in different parts of a tissue.

The term "sample", as used herein, refers to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, or cells there from. Biological samples may also include sections of tissues such as frozen or fixed sections taken for histological purposes or microdissected cells or extracellular parts thereof. A biological sample to be analyzed is tissue material from neoplastic lesion taken by aspiration or punctuation, excision or by any other surgical method leading to biopsy or resected cellular material. Such biological sample may comprise cells obtained from a patient. The cells may be found in a cell "smear" collected, for example, by a nipple aspiration, ductal lavarge, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

A "tumor sample" is a sample containing tumor material e.g. tissue material from a neoplastic lesion taken by aspiration or puncture, excision or by any other surgical method leading to biopsy or resected cellular material, including preserved material such as fresh frozen material, formalin fixed material, paraffin embedded material and the like. Such a biological sample may comprise cells obtained from a patient. The cells may be found in a cell "smear" collected, for example, by a nipple aspiration, ductal lavage, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

The term "mathematically combining expression levels", within the meaning of the invention shall be understood as deriving a numeric value from a determined expression level of a gene and applying an algorithm to one or more of such numeric values to obtain a combined numerical value or combined score.

A "score" within the meaning of the invention shall be understood as a numeric value, which is related to the outcome of a patient's disease and/or the response of a tumor to chemotherapy. The numeric value is derived by combining the expression levels of marker genes using pre-specified coefficients in a mathematic algorithm. The expression levels can be employed as CT or delta-CT values obtained by kinetic RT-PCR, as absolute or relative fluorescence intensity values obtained through microarrays or by any other method useful to quantify absolute or relative RNA levels. Combining these expression levels can be accomplished for example by multiplying each expression level with a defined and specified coefficient and summing up such products to yield a score. The score may be also derived from expression levels together with other information, e. g. clinical data like tumor size, lymph node status or tumor grading as such variables can also be coded as numbers in an equation. The score may be used on a continuous scale to predict the response of a tumor to chemotherapy and/or the outcome of a patient's disease. Cut-off values may be applied to distinguish clinical relevant subgroups. Cut-off values for such scores can be determined in the same way as cut-off values for conventional diagnostic markers and are well known to those skilled in the art. A useful way of determining such cut-off value is to construct a receiver-operator curve (ROC curve) on the basis of all conceivable cut-off values, determine the single point on the ROC curve with the closest proximity to the upper left corner (0/1) in the ROC plot. Obviously, most of the time cut-off values will be determined by less formalized procedures by choosing the combination of sensitivity and specificity determined by such cut-off value providing the most beneficial medical information to the problem investigated.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). Moreover, PCR-based methods comprise e.g. real time PCR, and, particularly suited for the analysis of expression levels, kinetic or quantitative PCR (qPCR).

A "microarray" herein also refers to a "biochip" or "biological chip", an array of regions having a density of discrete regions of at least about $100/cm^2$ , and preferably at least about $1000/cm^2$. The regions in a microarray have typical dimensions, e.g., diameters, in the range of between about 10-250 $\mu$m, and are separated from other regions in the array by about the same distance.

The term "hybridization-based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described above. When it comes to the determination of expression levels, hybridization based methods may for example be used to determine the amount of mRNA for a given gene.

The term "marker gene" as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and head and neck, colon or breast cancer in par-ticular. A marker gene may also have the characteristics of a target gene.

An "algorithm" is a process that performs some sequence of operations to produce information.

The term "measurement at a protein level", as used herein, refers to methods which allow the quantitative and/or qualitative determination of one or more proteins in a sample. These methods include, among others, protein purification, including ultracentrifugation, precipitation and chromatography, as well as protein analysis and determination, including immunohistochemistry, immunofluorescence, ELISA (enzyme linked immunoassay), RIA (radioimmunoassay) or the use of protein microarrays, two- hybrid screening, blotting methods including western blot, one- and two dimensional gelelectrophoresis, isoelectric focusing as well as methods being based on mass spectrometry like MALDI-TOF and the like.

The term "kinetic PCR" or "Quantitative PCR" (qPCR) refers to any type of a PCR method which allows the quantification of the template in a sample. Quantitative real-time PCR comprise different techniques of performance or product detection as for example the TaqMan technique or the LightCycler technique. The TaqMan technique, for examples, uses a dual-labelled fluorogenic probe. The TaqMan real-time PCR measures accumulation of a product via the fluorophore during the exponential stages of the PCR, rather than at the end point as in conventional PCR. The exponential increase of the product is used to determine the threshold cycle, CT, i.e. the number of PCR cycles at which a significant exponential increase in fluorescence is detected, and which is directly correlated with the number of copies of DNA template present in the reaction. The set up of the reaction is very similar to a conventional PCR, but is carried out in a real-time thermal cycler that allows measurement of fluorescent molecules in the PCR tubes. Different from regular PCR, in TaqMan real-time PCR a probe is added to the reaction, i.e., a single-stranded oligonucleotide complementary to a segment of 20-60 nucleotides within the DNA template and located between the two primers. A fluorescent reporter or fluorophore (e.g., 6-carboxyfluorescein, acronym: FAM, or tetrachlorofluorescin, acronym: TET) and quencher (e.g., tetramethylrhodamine, acronym: TAMRA, of dihydrocyclopyrroloindole tripeptide "minor groove binder", acronym: MGB) are covalently attached to the 5' and 3' ends of the probe , respectively [2]. The close proximity between fluorophore and quencher attached to the probe inhibits fluorescence from the fluorophore. During PCR, as DNA synthesis commences, the 5' to 3 ' exonuclease activity of the Taq polymerase degrades that proportion of the probe that has annealed to the template (Hence its name: Taq polymerase + PacMan) . Degradation of the probe releases the fluorophore from it and breaks the close proximity to the quencher, thus relieving the quenching effect and allowing fluorescence of the fluorophore. Hence, fluorescence detected in the real-time PCR thermal cycler is directly proportional to the fluorophore released and the amount of DNA template present in the PCR.

"Primer" and "probes", within the meaning of the invention, shall have the ordinary meaning of this term which is well known to the person skilled in the art of molecular biology. In a preferred embodiment of the invention "primer" and "probes" shall be understood as being polynucleotide molecules having a sequence identical, complementary, homologous, or homologous to the complement of regions of a target polynucleotide which is to be detected or quantified. In yet another embodiment nucleotide analogues and/or morpholinos are also comprised for usage as primers and/or probes. "Individually labeled probes", within the meaning of the invention, shall be understood as being molecular probes comprising a polynucleotide, oligonucleotide or nucleotide analogue and a label, helpful in the detection or quantification of the probe. Preferred labels are fluorescent molecules, luminescent molecules, radioactive molecules, enzymatic molecules and/or quenching molecules.

**Object of the Invention**

It is one object of the present invention to provide an improved method for the prediction of a response of a tumor in a patient suffering from or at risk of developing recurrent breast cancer - to taxane/anthracycline-containing chemotherapy.

It is another object of the present invention to avoid unnecessary adjuvant and/or neoadjuvant cytotoxic chemotherapy in patients suffering especially breast cancer.

It is another disclosure to offer a more robust and specific diagnostic assay system than conventional immunohistochemistry for clinical routine fixed tissue samples that better helps the physician to select individualized treatment modalities.

In a more preferred embodiment the disclosed method can be used to select a suitable therapy for a neoplastic disease, particularly breast cancers.

It is another disclosure to detect new targets for newly available targeted drugs, or to determine drugs yet to be developed.

## Summary of the Invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a, ""an" and "the" include singular and/or plural referents unless the context clearly dictates otherwise. It is moreover to be understood that, in case parameter ranges are given which are delimited by numeric values, the ranges are deemed to include these limitation values.

The above problems are solved by methods provided by the invention.

Estrogen receptor status is generally determined using immunohistochemistry. HER2/NEU (ERBB2) status is generally determined using immunohistochemistry and fluorescence in situ hybridization. However, estrogen receptor status and HER2/NEU (ERBB2) status may, for the purposes of the invention, be determined by any suitable method, e.g. immunohistochemistry, fluorescence in situ hybridization (FISH), or gene expression analysis.

The present invention relates to a method for predicting a response to and/or benefit of taxane/anthracy ine-containing chemotherapy including neoadjuvant chemotherapy as defined in claim 1.

WO 2011/120984A1 utilizes the nine genes, however, for predicting an outcome of breast cancer in an estrogen receptor positive and HER2 negative tumor of a breast cancer patient, which is not related with the method of the present invention which is predicting a response to/or benefit of chemotherapy. The genes of the present invention are used for a different aim.

In one disclosure the method comprises:

(a) determining in a tumor sample from said patient the RNA expression levels of the following 8 genes: UBE2C, RACGAP1, DHCR7, STC2, AZGP1, RBBP8, IL6ST, and MGP, indicative of a response to chemotherapy for a tumor
(b) mathematically combining expression level values for the genes of the said set which values were determined in the tumor sample to yield a combined score, wherein said combined score is predicting said response and/or benefit of chemotherapy.

In a further disclosure the method of the invention comprises:

(a) determining in a tumor sample from said patient the RNA expression levels of the following 8 genes: UBE2C, BIRC5, DHCR7, STC2, AZGP1, RBBP8, IL6ST, and MGP; indicative of a response to chemotherapy for a tumor
**while BIRC5** may be replaced by UBE2C or TOP2A or RACGAP1 or AURKA or NEK2 or E2F8 or PCNA or CYBRD1 or DCN or ADRA2A or SQLE or CXCL12 or EPHX2 or ASPH or PRSS16 or EGFR or CCND1 or TRIM29 or DHCR7 or PIP or TFAP2B or WNT5A or APOD or PTPRT with the proviso that after a replacement 8 different genes are selected; and
**while UBE2C** may be replaced by BIRC5 or RACGAP1 or TOP2A or AURKA or NEK2 or E2F8 or PCNA or CYBRD1 or ADRA2A or DCN or SQLE or CCND1 or ASPH or CXCL12 or PIP or PRSS16 or EGFR or DHCR7 or EPHX2 or TRIM29 with the proviso that after a replacement 8 different genes are selected; and
**while DHCR7** may be replaced by AURKA, BIRC5, UBE2C or by any other gene that may replace BIRC5 or UBE2C with the proviso that after a replacement 8 different genes are selected; and
**while STC2** may be replaced by INPP4B or IL6ST or SEC14L2 or MAPT or CHPT1 or ABAT or SCUBE2 or ESR1 or RBBP8 or PGR or PTPRT or HSPA2 or PTGER3 with the proviso that after a replacement 8 different genes are selected; and
**while AZGP1** may be replaced by PIP or EPHX2 or PLAT or SEC14L2 or SCUBE2 or PGR with the proviso that after a replacement 8 different genes are selected; and
**while RBBP8** may be replaced by CELSR2 or PGR or STC2 or ABAT or IL6ST with the proviso that after a replacement 8 different genes are selected; and
**while IL6ST** may be replaced by INPP4B or STC2 or MAPT or SCUBE2 or ABAT or PGR or SEC14L2 or ESR1 or GJA1 or MGP or EPHX2 or RBBP8 or PTPRT or PLAT with the proviso that after a replacement 8 different genes are selected; and
**while MGP** may be replaced by APOD or IL6ST or EGFR with the proviso that after a replacement 8 different genes are selected;

(b) mathematically combining expression level values for the genes of the said set which values were determined in the tumor sample to yield a combined score, wherein said combined score is predicting said response and/or benefit of chemotherapy.

The methods of the invention are suited for predicting a response to taxane/anthracycline-containing chemotherapy, preferably in Her2/neu negative, estrogen receptor positive (luminal) tumors, preferably in the neodadjuvant mode.

According to an aspect of the disclosure there is provided a method as described above, wherein said expression level is determined as a mRNA level. According to an aspect of the invention there is provided a method as described above, wherein said expression level is determined as a gene expression level.

According to an aspect of the invention there is provided a method as described above, wherein said expression level is determined by at least one of

a PCR based method,

a microarray based method,

a hybridization based method, and.

a sequencing and/or next generation sequencing approach

According to an aspect of the invention there is provided a method as described above, wherein said determination of expression levels is in a formalin-fixed paraffin-embedded tumor sample or in a fresh-frozen tumor sample.

According to an aspect of the invention there is provided a method as described above, wherein the expression level of said at least one marker gene is determined as a pattern of expression relative to at least one reference gene or to a computed average expression value.

According to an aspect of the invention there is provided a method as described above, wherein said step of mathematically combining comprises a step of applying an algorithm to values representative of an expression level of a given gene.

According to an aspect of the invention there is provided a method as described above, wherein said algorithm is a linear combination of said values representative of an expression level of a given gene.

According to an aspect of the invention there is provided a method as described above, wherein a value for a representative of an expression level of a given gene is multiplied with a coefficient.

According to an aspect of the invention there is provided a method as described above, wherein one, two or more thresholds are determined for said combined score and discriminated into high and low risk, high, intermediate and low risk, or more risk groups by applying the threshold on the combined score.

According to an aspect of the invention there is provided a method as described above, wherein a high combined score is indicative of benefit from a more aggressive therapy, e.g. cytotoxic chemotherapy. The skilled person understands that a "high score" in this regard relates to a reference value or cut-off value. The skilled person further understands that depending on the particular algorithm used to obtain the combined score, also a "low" score below a cut off or reference value can be indicative of benefit from a more aggressive therapy, e.g. cytotoxic chemotherapy.

According to an aspect of the invention there is provided a method as described above, wherein information regarding nodal status of the patient is processed in the step of mathematically combining expression level values for the genes to yield a combined score.

According to an aspect of the invention there is provided a method as described above, wherein said information regarding nodal status is a numerical value $\leq 0$ if said nodal status is negative and said information is a numerical value $> 0$ if said nodal status positive or unknown. In exemplary embodiments of the invention a negative nodal status is assigned the value 0, an unknown nodal status is assigned the value 0.5 and a positive nodal status is assigned the value 1. Other values may be chosen to reflect a different weighting of the nodal status within an algorithm.

According to an aspect of the invention there is provided a method as described above, wherein said information regarding tumor size of the patient is processed in the step of mathematically combining expression level values for the genes to yield a combined score.

According to an aspect of the invention there is provided a method as described above, wherein said information regarding nodal status and tumor size of the patient is processed in the step of mathematically combining expression level values for the genes to yield a combined score.

The disclosure further relates to a kit for performing a method as described above, said kit comprising a set of oligonucleotides capable of specifically binding sequences or to sequences of fragments of the genes in a combination of genes, wherein

(i) said combination comprises at least the 8 genes UBE2C, BIRC5, DHCR7, STC2, AZGP1, RBBP8, IL6ST, and MGP; or

(ii) said combination comprises at least the 8 genes UBE2C, RACGAP1, DHCR7, STC2, AZGP1, RBBP8, IL6ST, and MGP.

The disclosure further relates to a computer program product capable of processing values representative of an expression level of a combination of genes mathematically combining said values to yield a combined score, wherein said combined score is predicting said response and/or benefit of chemotherapy of said patient.

Said computer program product may be stored on a data carrier or implemented on a diagnostic system capable of outputting values representative of an expression level of a given gene, such as a real time PCR system.

If the computer program product is stored on a data carrier or running on a computer, operating personal can input the expression values obtained for the expression level of the respective genes. The computer program product can then apply an algorithm to produce a combined score indicative of benefit from cytotoxic chemotherapy for a given patient.

The methods of the present invention have the advantage of providing a reliable prediction of response and/or benefit of chemotherapy based on the use of only a small number of genes. The methods of the present invention have been found to be especially suited for analyzing the response and/or benefit of chemotherapy of patients with tumors classified as ESR1 positive and ERBB2 negative.

**Detailed description of the invention**

Additional details, features, characteristics and advantages of the object of the invention are disclosed in the sub-claims, and the following description of the respective figures and examples, which, in an exemplary fashion, show preferred embodiments of the present invention. However, these drawings should by no means be understood as to limit the scope of the invention.

Four public available gene expression data sets (Affymetrix HG-U133A) were retrieved from the gene expression omnibus (GEO) data repository. All analyzed breast cancer patients were treated with anthracycline or taxan/anthracycline-based neoadjuvant chemotherapy. Microarray cell files were MAS5 normalized with a global scaling procedure and a target intensity of 500. Pathological complete response (pCR) was used as the primary endpoint for the assessment of treatment response. The analysis was performed in all HER2/neu-negative breast cancer patients and in the subset of ER-positive, HER2-negative breast cancer patients according to pre-specified cut-off levels (ERBB2 probeset 216836 < 6000 = HER2/neu-negative, ERBB2 probeset 216836 < 6000 and ESR1 probeset > 1000 = ER-positive/HER2/neu-negative).

The T5 score was examined in 374 HER2-negative breast cancer patients treated with neoadjuvant therapy (figure 1). Among the 374 patients, 63 tumors (16.8 %) were classified as T5-low-risk, whereas 311 tumors (83.2%) were T5-high-risk. Only one of the T5-low-risk tumors achieved a pCR after neoadjuvant therapy, whereas 84 of the 85 pCR events were classified as T5-high risk. The sensitivity of the T5 score was 99% and the negative predictive value 98% with an area under the receiver operating characteristic curve of 0.69(figure 1).

The Figure 1 shows:

a) T5 score distribution in 374 HER2/neu-negative breast cancer patients (85 pCR events vs. 289 samples with residual disease); two-sided Mann-Whitney Test

b) Using the pre-specified cut-off T5 score 5, the sensitivity was 99%, the specificity 21%, the negative predictive value 98% and the positive predictive value 27% with an area under the receiver operating curve of 0.69.

The T5 score was examined in 221 ER-positive, HER2-negative breast cancer patients treated with neoadjuvant therapy (figure 2). Among the 221 patients, 61 tumors (27.6 %) were classified as T5-low-risk, whereas 160 tumors (72.4%) were T5-high-risk. Only one of the T5-low-risk tumors achieved a pCR after neoadjuvant therapy, whereas 24 of the 25 pCR events were classified as T5-high risk. The sensitivity of the T5 score was 96% and the negative predictive value 98% with an area under the receiver operating characteristic curve of 0.73 (figure 2).

The Figure 2 shows:

c) T5 score distribution in 221 estrogen receptor positive and HER2/neu-negative breast cancer patients (25 pCR events vs. 196 samples with residual disease); two-sided Mann-Whitney Test

d) Using the pre-specified cut-off T5 score 5, the sensitivity was 96%, the specificity 30%, the negative predictive value 98% and the positive predictive value 15% with an area under the receiver operating curve of 0.73.

Herein disclosed are unique combinations of marker genes which can be combined into an algorithm for the here presented new predictive test. Technically, the method of the invention can be practiced using two technologies: 1.) Isolation of total RNA from fresh or fixed tumor tissue and 2.) Quantitative RT-PCR of the isolated nucleic acids. Alternatively, it is contemplated to measure expression levels using alternative technologies, e.g by microarray, in particular affymetrix U-133 arrays or by measurement at a protein level.

The methods of the invention are based on quantitative determination of RNA species isolated from the tumor in order to obtain expression values and subsequent bioinformatic analysis of said determined expression values. RNA species can be isolated from any type of tumor sample, e.g. biopsy samples, smear samples, resected tumor material, fresh frozen tumor tissue or from paraffin embedded and formalin fixed tumor tissue. First, RNA levels of genes coding for specific combinations of the genes UBE2C, BIRC5, DHCR7, RACGAP1, AURKA, PVALB, NMU, STC2, AZGP1, RBBP8, IL6ST, MGP, PTGER3, CXCL12, ABAT, CDH1, and PIP or specific combinations thereof, as indicated, are determined. Based on these expression values a predictive score is calculated by a mathematical combination, e.g. according to formulas T5, T1, T4, or T5b (see below).

A high score value indicates an increased likelihood of a pathological complete response after neoadjuvant chemotherapy treatment, a low score value indicates a decreased likelihood of developing a pathological complete response after neoadjuvant treatment. Consequently, a high score also indicates that the patient is a high risk patient who will benefit from a more aggressive therapy, e.g. cytotoxic chemotherapy.

Table 1, below, shows the combinations of genes used for each algorithm.

Table 1: Combination of genes for the respective algorithms:

| Gene | Algo_T1 | Algo_T4 | Algo_T5 | Algo_T5b |
|---|---|---|---|---|
| UBE2C | | | X | |
| BIRC5 | X | X | X | |
| DHCR7 | | X | X | X |
| RACGAP1 | | X | | X |
| AURKA | X | | | |
| PVALB | X | X | | |
| NMU | X | | | X |
| STC2 | X | X | X | |
| AZGP1 | | | X | X |
| RBBP8 | X | | X | X |
| IL6ST | | X | X | X |
| MGP | | | X | X |
| PTGER3 | X | X | | |
| CXCL12 | X | X | | |
| ABAT | | X | | |
| CDH1 | X | | | |
| PIP | X | | | |

Table 2, below, shows Affy probeset ID and TaqMan design ID mapping of the marker genes of the present invention.

Table 2: Gene symbol, Affy probeset ID and TaqMan design ID mapping:

| Gene | Design ID | Probeset ID |
|---|---|---|
| UBE2C | R65 | 202954_at |
| BIRC5 | SC089 | 202095_s_at |

(continued)

| Gene | Design ID | Probeset ID |
|---|---|---|
| DHCR7 | CAGMC334 | 201791_s_at |
| RACGAP1 | R125-2 | 222077_s_at |
| AURKA | CAGMC336 | 204092_s_at |
| PVALB | CAGMC339 | 205336_at |
| NMU | CAGMC331 | 206023_at |
| STC2 | R52 | 203438_at |
| AZGP1 | CAGMC372 | 209309_at |
| RBBP8 | CAGMC347 | 203344_s_at |
| IL6ST | CAGMC312 | 212196_at |
| MGP | CAGMC383 | 202291_s_at |
| PTGER3 | CAGMC315 | 213933_at |
| CXCL12 | CAGMC342 | 209687_at |
| ABAT | CAGMC338 | 209460_at |
| CDH1 | CAGMC335 | 201131_s_at |

Table 3, below, shows full names, Entrez GeneID, gene bank accession number and chromosomal location of the marker genes of the present invention

| Official Symbol | Official Full Name | Entrez GeneID | Accesion Number | Location |
|---|---|---|---|---|
| UBE2C | ubiquitin-conjugating enzyme E2C | 11065 | U73379 | 20q13. 12 |
| BIRC5 | baculoviral IAP repeat-containing 5 | 332 | U75285 | 17q25 |
| DHCR7 | 7-dehydrocholesterol reductase | 1717 | AF034544 | 11q13. 4 |
| STC2 | staniocalcin 2 | 8614 | AB012664 | 5q35.2 |
| RBBP8 | retinoblastoma binding protein 8 | 5932 | AF043431 | 18q11. 2 |
| IL6ST | interleukin 6 signal transducer | 3572 | M57230 | 5q11 |
| MGP | matrix Gla protein | 4256 | M58549 | 12p12. 3 |
| AZGP1 | alpha-2-glycoprotein 1, zinc-binding | 563 | BC005306 | 11q22. 1 |
| RACGAP1 | Rac GTPase activating protein 1 | 29127 | NM_013277 | 12q13 |
| AURKA | aurora kinase A | 6790 | BC001280 | 20q13 |
| PVALB | parvalbumin | 5816 | NM_002854 | 22q13. 1 |
| NMU | neuromedin U | 10874 | X76029 | 4q12 |
| PTGER3 | prostaglandin E receptor 3 (subtype EP3) | 5733 | X83863 | 1p31.2 |
| CXCL12 | chemokine (C-X-C motif) ligand 12 (stromal cell-derived factor 1) | 6387 | L36033 | 10q11. 1 |
| ABAT | 4-aminobutyrat aminotransferase | 18 | L32961 | 16p13. 2 |
| CDH1 | cadherin 1, type 1, E-cadherin (epithelial) | 999 | L08599 | 16q22. 1 |
| PIP | prolactin-induced protein | 5304 | NMM_002652 | 7q32-qter |

Example algorithm T5:

Algorithm T5 is a committee of four members where each member is a linear combination of two genes. The mathematical formulas for T5 are shown below; the notation is the same as for T1. T5 can be calculated from gene expression data only.

riskMember1 = 0.434039 [0.301..0.567] * (0.939 * BIRC5 -3.831)

-0.491845 [-0.714..-0.270] * (0.707 * RBBP8 -0.934)

riskMember2 = 0.488785 [0.302..0.675] * (0.794 * UBE2C -1.416)

-0.374702 [-0.570..-0.179] * (0.814 * IL6ST -5.034)

riskMember3 = -0.39169 [-0.541..-0.242] * (0.674 * AZGP1 -0.777)

+0.44229 [0.256..0.628] * (0.891 * DHCR7 -4.378)

riskMember4 = -0.377752 [-0.543..-0.212] * (0.485 * MGP +4.330)

-0.177669 [-0.267..-0.088] * (0.826 * STC2 -3.630)

risk = riskMember1 + riskMember2 + riskMember3 + riskMember4

Coefficients on the left of each line were calculated as COX proportional hazards regression coefficients, the numbers in squared brackets denote 95% confidence bounds for these coefficients. In other words, instead of multiplying the term (0.939 * BIRC5 -3.831) with 0.434039, it may be multiplied with any coefficient between 0.301 and 0.567 and still give a predictive result with in the 95% confidence bounds. Terms in round brackets on the right of each line denote a platform transfer from PCR to Affymetrix: The variables PVALB, CDH1, ... denote PCR-based expressions normalized by the reference genes (delta-Ct values), the whole term within round brackets corresponds to the logarithm (base 2) of Affymetrix microarray expression values of corresponding probe sets.

Example algorithm T5clin:

Algorithm T5clin is a combined score consisting of the T5 score and clinical parameters (nodal status and tumor size).

$$T5clin = 0.35 * t + 0.64 * n + 0.28*s$$

where *t* codes for tumor size (1: ≤1 cm, 2: >1 cm to ≤2 cm, 3: >2 cm to ≤5 cm, 4: >5 cm), and *n* for nodal status (1: negative, 2: 1 to 3 positive nodes, 3: 4 to 10 positive nodes, 4: >10 positive nodes).

In a preferred in embodiment, the threshold for the T5clin score is 3.3.

Example algorithm T1:

Algorithm T1 is a committee of three members where each member is a linear combination of up to four variables. In general variables may be gene expressions or clinical variables. In T1 the only non-gene variable is the nodal status coded 0, if patient is lymph-node negative and 1, if patient is lymph-node-positive. The mathematical formulas for T1 are shown below.

riskMember1 = +0.193935 [0.108..0.280] * (0.792 * PVALB -2.189)
-0.240252 [-0.400..-0.080] * (0.859 * CDH1 -2.900)
-0.270069 [-0.385..-0.155] * (0.821 * STC2 -3.529)
+1.2053 [0.534..1.877] * nodalStatus

riskMember2 = -0.25051 [-0.437..-0.064] * (0.558 * CXCL12 +0.324)
-0.421992 [-0.687..-0.157] * (0.715 * RBBP8 -1.063)
+0.148497 [0.029..0.268] * (1.823 * NMU -12.563)
+0.293563 [0.108..0.479] * (0.989 * BIRC5 -4.536)

riskMember3 = +0.308391 [0.074..0.543] * (0.812 * AURKA -2.656)
-0.225358 [-0.395..-0.055] * (0.637 * PTGER3 + 0.492)
-0.116312 [-0.202..-0.031] * (0.724 * PIP + 0.985)

risk = + riskMember1 + riskMember2 + riskMember3

Coefficients on the left of each line were calculated as COX proportional hazards regression coefficients, the numbers in squared brackets denote 95% confidence bounds for these coefficients. Terms in round brackets on the right of each line denote a platform transfer from PCR to Affymetrix: The variables PVALB, CDH1, ... denote PCR-based expressions normalized by the reference genes, the whole term within round brackets corresponds to the logarithm (base 2) of Affymetrix microarray expression values of corresponding probe sets.

Example algorithm T4:

Algorithm T4 is a linear combination of motifs. The top 10 genes of several analyses of Affymetrix datasets and PCR data were clustered to motifs. Genes not belonging to a cluster were used as single gene-motifs. COX proportional hazards regression coefficients were found in a multivariate analysis.

In general motifs may be single gene expressions or mean gene expressions of correlated genes. The mathematical formulas for T4 are shown below.

prolif = ((0.84 [0.697..0.977] * RACGAP1 -2.174) + (0.85 [0.713..0.988] *DHCR7 -3.808)+ (0.94 [0.786..1.089] * BIRC5 -3.734)) / 3

motiv2 = ((0.83 [0.693..0.96] * IL6ST -5.295) + (1.11 [0.930..1.288] * ABAT -7.019) + (0.84 [0.701..0.972] * STC2 -3.857)) / 3

ptger3 = (PTGER3 * 0.57 [0.475..0.659] + 1.436)

cxcl12 = (CXCL12 * 0.53 [0.446..0.618] + 0.847)

pvalb = (PVALB * 0.67 [0.558..0.774] -0.466)

Factors and offsets for each gene denote a platform transfer from PCR to Affymetrix: The variables RACGAP1, DHCR7, ... denote PCR-based expressions normalized by CALM2 and PPIA, the whole term within round brackets corresponds to the logarithm (base 2) of Affymetrix microarray expression values of corresponding probe sets.

The numbers in squared brackets denote 95% confidence bounds for these factors.

As the algorithm performed even better in combination with a clinical variable the nodal status was added. In T4 the nodal status is coded 0, if patient is lymph-node negative and 1, if patient is lymph-node-positive. With this, algorithm T4 is:

$$\begin{aligned} risk = &-0.32\ [-0.510..-0.137] * motiv2 \\ &+ 0.65\ [0.411..0.886] * prolif \\ &- 0.24\ [-0.398..-0.08] * ptger3 \\ &- 0.05\ [-0.225..0.131] * cxcl12 \\ &+ 0.09\ [0.019..0.154] * pvalb \\ &+ nodalStatus \end{aligned}$$

Coefficients of the risk were calculated as COX proportional hazards regression coefficients, the numbers in squared brackets denote 95% confidence bounds for these coefficients.

Algorithm T5b is a committee of two members where each member is a linear combination of four genes. The mathematical formulas for T5b are shown below, the notation is the same as for T1 and T5. In T5b a non-gene variable is the nodal status coded 0, if patient is lymph-node negative and 1, if patient is lymph-node-positive and 0.5 if the lymph-node status is unknown. T5b is defined by:

$$\begin{aligned} riskMember1 = &\ 0.359536\ [0.153..0.566] * (0.891 * DHCR7 -4.378) \\ &-0.288119\ [-0.463..-0.113] * (0.485 * MGP + 4.330) \\ &+0.257341\ [0.112..0.403] * (1.118 * NMU -5.128) \\ &-0.337663\ [-0.499..-0.176] * (0.674 * AZGP1 -0.777) \end{aligned}$$

$$\begin{aligned} riskMember2 = &-0.374940\ [-0.611..-0.139] * (0.707 * RBBP8 -0.934) \\ &-0.387371\ [-0.597..-0.178] * (0.814 * IL6ST -5.034) \\ &+0.800745\ [0.551..1.051] * (0.860 * RACGAP1 -2.518) \\ &+0.770650\ [0.323..1.219] * Nodalstatus \end{aligned}$$

$$risk = riskMember1 + riskMember2$$

The skilled person understands that these algorithms represent particular examples and that based on the information regarding association of gene expression with the prediction of therapeutic response.

Algorithm Simplification by employing Subsets of Genes

"Example algorithm T5" is a committee predictor consisting of 4 members with 2 genes of interest each. Each member is an independent and self-contained predictor of distant recurrence and/or therapy response, each additional member contributes to robustness and predictive power of the algorithm. The equation below shows the "Example Algorithm T5"; for ease of reading the number of digits after the decimal point has been truncated to 2; the range in square brackets lists the estimated range of the coefficients (mean +/- 3 standard deviations).

T5 Algorithm:

+0.41 [0.21..0.61] * BIRC5 -0.33 [-0.57..-0.09] * RBBP8
+0.38 [0.15..0.61] * UBE2C -0.30 [-0.55..-0.06] * IL6ST
-0.28 [-0.43..-0.12] * AZGP1 +0.42 [0.16..0.68] * DHCR7
-0.18 [-0.31..-0.06] * MGP -0.13 [-0.25..-0.02] * STC2

c-indices: trainSet=0.724,

Gene names in the algorithm denote the difference of the mRNA expression of the gene compared to one or more housekeeping genes as described above.

Analyzing a cohort different from the finding cohort (234 tumor samples) it was surprising to learn that some simplifications of the "original T5 Algorithm" still yielded a diagnostic performance not significantly inferior to the original T5 algorithm. The most straightforward simplification was reducing the committee predictor to one member only. Examples for the performance of the "one-member committees" are shown below:

member 1 only:

+0.41 [0.21..0.61] * BIRC5 -0.33 [-0.57..-0.09] * RBBP8

c-indices: trainSet=0.653, independentCohort=0.681

member 2 only:

+0.38 [0.15..0.61] * UBE2C -0.30 [-0.55..-0.06] * IL6ST

c-indices: trainSet=0.664, independentCohort=0.696

member 3 only:

-0.28 [-0.43..-0.12] * AZGP1 +0.42 [0.16..0.68] * DHCR7

c-indices: trainSet=0.666, independentCohort=0.601

member 4 only:

-0.18 [-0.31..-0.06] * MGP -0.13 [-0.25..-0.02] * STC2

c-indices: trainSet=0.668, independentCohort=0.593

The performance of the one member committees as shown in an independent cohort of 234 samples is notably reduced compared to the performance of the full algorithm.

Gradually combining more than one but less than four members to a new prognostic committee predictor algorithm, frequently leads to a small but significant increase in the diagnostic performance compared to a one-member committee. It was surprising to learn that there were marked improvements by some combination of committee members while other combinations yielded next to no improvement. Initially, the hypothesis was that a combination of members representing similar biological motives as reflected by the employed genes yielded a smaller improvement than combining members reflecting distinctly different biological motives. Still, this was not the case. No rule could be identified to foretell the combination of some genes to generate an algorithm exhibiting more prognostic power than another combination of genes. Promising combinations could only be selected based on experimental data. Identified combinations of combined committee members to yield simplified yet powerful algorithms are shown below.
members 1 and 2 only:

+0.41 [0.21..0.61] * BIRC5 -0.33 [-0.57..-0.09] * RBBP8
+0.38 [0.15..0.61] * UBE2C -0.30 [-0.55..-0.06] * IL6ST

c-indices: trainSet=0.675, independentCohort=0.712
members 1 and 3 only:

+0.41 [0.21..0.61] * BIRC5 -0.33 [-0.57..-0.09] * RBBP8
-0.28 [-0.43..-0.12] * AZGP1 +0.42 [0.16..0.68] * DHCR7

c-indices: trainSet=0.697, independentCohort=0.688
members 1 and 4 only:

+0.41 [0.21..0.61] * BIRC5 -0.33 [-0.57..-0.09] * RBBP8
-0.18 [-0.31..-0.06] * MGP -0.13 [-0.25..-0.02] * STC2

c-indices: trainSet=0.705, independentCohort=0.679
members 2 and 3 only:

+0.38 [0.15..0.61] * UBE2C -0.30 [-0.55..-0.06] * IL6ST

-0.28 [-0.43..-0.12] * AZGP1 +0.42 [0.16..0.68] * DHCR7

c-indices: trainSet=0.698, independentCohort=0.670
members 1, 2 and 3 only:

+0.41 [0.21..0.61] * BIRC5 -0.33 [-0.57..-0.09] * RBBP8
+0.38 [0.15..0.61] * UBE2C -0.30 [-0.55..-0.06] * IL6ST
-0.28 [-0.43..-0.12] * AZGP1 +0.42 [0.16..0.68] * DHCR7

c-indices: trainSet=0.701, independentCohort=0.715

Not omitting complete committee members but a single gene or genes from different committee members is also possible but requires a retraining of the entire algorithm. Still, it can also be advantageous to perform. The performance of simplified algorithms generated by omitting entire members or individual genes is largely identical.

Algorithm Variants by Gene Replacement

Described algorithms, such as "Example algorithm T5", above can be also be modified by replacing one or more genes by one or more other genes. The purpose of such modifications is to replace genes difficult to measure on a specific platform by a gene more straightforward to assay on this platform. While such transfer may not necessarily yield an improved performance compared to a starting algorithm, it can yield the clue to implanting the prognostic algorithm to a particular diagnostic platform. In general, replacing one gene by another gene while preserving the diagnostic power of the predictive algorithm can be best accomplished by replacing one gene by a co-expressed gene with a high correlation (shown e.g. by the Pearson correlation coefficient). Still, one has to keep in mind that the mRNA expression of two genes highly correlative on one platform may appear quite independent from each other when assessed on another platform. Accordingly, such an apparently easy replacement when reduced to practice experimentally may yield disappointingly poor results as well as surprising strong results, always depending on the imponderabilia of the platform employed. By repeating this procedure one can replace several genes.

The efficiency of such an approach can be demonstrated by evaluating the predictive performance of the T5 algorithm score and its variants on the validation cohorts. The following table shows the c-index with respect to endpoint distant recurrence in two validation cohorts.

| Variant | Validation Study A | Validation Study B |
|---|---|---|
| original algorithm T5 | c-index = 0.718 | c-index = 0.686 |
| omission of BIRC5 (setting expression to some constant) | c-index = 0.672 | c-index = 0.643 |
| replacing BIRC5 by UBE2C (no adjustment of the coefficient) | c-index = 0.707 | c-index = 0.678 |

One can see that omission of one of the T5 genes, here shown for BIRC5 for example, notably reduces the predictive performance. Replacing it with another gene yields about the same performance.

A better method of replacing a gene is to re-train the algorithm. Since T5 consists of four independent committee members one has to re-train only the member that contains the replaced gene. The following equations demonstrate replacements of genes of the T5 algorithm shown above trained in a cohort of 234 breast cancer patients. Only one member is shown below, for c-index calculation the remaining members were used unchanged from the original T5 Algorithm. The range in square brackets lists the estimated range of the coefficients: mean +/- 3 standard deviations.

Member 1 of T5:

Original member 1:

+0.41 [0.21..0.61] * BIRC5 -0.33 [-0.57..-0.09] * RBBP8

c-indices: trainSet=0.724, independentCohort=0.705
replace BIRC5 by TOP2A in member 1:

+0.47 [0.24..0.69] * TOP2A -0.34 [-0.58..-0.10] * RBBP8

c-indices: trainSet=0.734, independentCohort=0.694
replace BIRC5 by RACGAP1 in member 1:

+0.69 [0.37..1.00] * RACGAP1 -0.33 [-0.57..-0.09] * RBBP8

c-indices: trainSet=0.736, independentCohort=0.743
replace RBBP8 by CELSR2 in member 1:

+0.38 [0.19..0.57] * BIRC5 -0.18 [-0.41..0.05] * CELSR2

c-indices: trainSet=0.726, independentCohort=0.680
replace RBBP8 by PGR in member 1:

+0.35 [0.15..0.54] * BIRC5 -0.09 [-0.23..0.05] * PGR

c-indices: trainSet=0.727, independentCohort=0.731

Member 2 of T5:

Original member 2:

+0.38 [0.15..0.61] * UBE2C -0.30 [-0.55..-0.06] * IL6ST

c-indices: trainSet=0.724, independentCohort=0.725
replace UBE2C by RACGAP1 in member 2:

+0.65 [0.33..0.96] * RACGAP1 -0.38 [-0.62..-0.13] * IL6ST

c-indices: trainSet=0.735, independentCohort=0.718
replace UBE2C by TOP2A in member 2:

+0.42 [0.20..0.65] * TOP2A -0.38 [-0.62..-0.13] * IL6ST

c-indices: trainSet=0.734, independentCohort=0.700
replace IL6ST by INPP4B in member 2:

+0.40 [0.17..0.62] * UBE2C -0.25 [-0.55..0.05] * INPP4B

c-indices: trainSet=0.725, independentCohort=0.686
replace IL6ST by MAPT in member 2:

+0.45 [0.22..0.69] * UBE2C -0.14 [-0.28..0.01] * MAPT

c-indices: trainSet=0.727, independentCohort=0.711

Member 3 of T5:

Original member 3:

-0.28 [-0.43..-0.12] * AZGP1 +0.42 [0.16..0.68] * DHCR7

c-indices: trainSet=0.724, independentCohort=0.705
replace AZGP1 by PIP in member 3:

-0.10 [-0.18..-0.02] * PIP +0.43 [0.16..0.70] * DHCR7

c-indices: trainSet=0.725, independentCohort=0.692
replace AZGP1 by EPHX2 in member 3:

-0.23 [-0.43..-0.02] * EPHX2 +0.37 [0.10..0.64] * DHCR7

c-indices: trainSet=0.719, independentCohort=0.698
replace AZGP1 by PLAT in member 3:

-0.23 [-0.40..-0.06] * PLAT +0.43 [0.18..0.68] * DHCR7

c-indices: trainSet=0.712, independentCohort=0.715

replace DHCR7 by AURKA in member 3:

$$-0.23\ [-0.39..-0.06] * AZGP1 +0.34\ [0.10..0.58] * AURKA$$

c-indices: trainSet=0.716, independentCohort=0.733

Member 4 of T5:

Original member 4:

$$-0.18\ [-0.31..-0.06] * MGP -0.13\ [-0.25..-0.02] * STC2$$

c-indices: trainSet=0.724, independentCohort=0.705
replace MGP by APOD in member 4:

$$-0.16\ [-0.30..-0.03] * APOD -0.14\ [-0.26..-0.03] * STC2$$

c-indices: trainSet=0.717, independentCohort=0.679
replace MGP by EGFR in member 4:

$$-0.21\ [-0.37..-0.05] * EGFR -0.14\ [-0.26..-0.03] * STC2$$

c-indices: trainSet=0.715, independentCohort=0.708
replace STC2 by INPP4B in member 4:

$$-0.18\ [-0.30..-0.05] * MGP -0.22\ [-0.53..0.08] * INPP4B$$

c-indices: trainSet=0.719, independentCohort=0.693
replace STC2 by SEC14L2 in member 4:

$$-0.18\ [-0.31..-0.06] * MGP -0.27\ [-0.49..-0.06] * SEC14L2$$

c-indices: trainSet=0.718, independentCohort=0.681

One can see that replacements of single genes experimentally identified for a quantification with quantitative PCR normally affect the predictive performance of the T5 algorithm, assessed by the c-index only insignificantly.

The following table shows potential replacement gene candidates for the genes of T5 algorithm. Each gene candidate is shown in one table cell: The gene name is followed by the bracketed absolute Pearson correlation coefficient of the expression of the original gene in the T5 Algorithm and the replacement candidate, and the HG-U133A probe set ID.

| BIRC5 | RBBP8 | UBE2C | IL6ST | AZGP1 | DHCR7 | MGP | STC2 |
|---|---|---|---|---|---|---|---|
| UBE2C (0.775), 202954_at | CELSR2 (0.548), 204029_at | BIRC5 (0.775), 202095_s_at | INPP4B (0.477), 205376_at | PIP (0.530), 206509_at | AURKA (0.345), 204092_s_at | APOD (0.368), 201525_at | INPP4B (0.500), 205376_at |
| TOP2A (0.757), 201292_at | PGR(0.392), 208305_at | RACGAP1 (0.756), | STC2 (0.450), 203438_at | EPHX2 (0.369), 209368_at | BIRC5 (0.323), 202095_s_at | IL6ST (0.327), 212196_at | IL6ST (0.450), 212196_at |
| RACGAP1 (0.704), | STC2 (0.361), 203438_at | TOP2A (0.753), 201292_at | MAPT (0.440), 206401_s_at | PLAT (0.366), 201860_s_at | UBE2C (0.315), 202954_at | EGFR (0.308), 201983_s_at | SEC14L2 (0.417), 204541_at |
| AURKA (0.681), 204092_s_at | ABAT (0.317), 209459_s_at | AURKA (0.694), 204092_s_at | SCUBE2 (0.418), 219197_s_at | SEC14L2 (0.351), 204541_at | | | MAPT (0.414), 206401_s_at |
| NEK2 (0.680), 204026_s_at | IL6ST (0.311), 212196_at | NEK2 (0.684), 204026_s_at | ABAT (0.389), 209459_s_at | SCUBE2 (0.331), 219197_s_at | | | CHPT1 (0.410), 221675_s_at |
| E2F8 (0.640), 219990_at | | E2F8 (0.652), 219990_at | PGR (0.377), 208305_at | PGR (0.302), 208305_at | | | ABAT (0.409), 209459_s_at |
| PCNA (0.544), 201202_at | | PCNA (0.589), 201202_at | SEC14L2 (0.356), 204541_at | | | | SCUBE2 (0.406), 219197_s_at |
| CYBRD1 (0.462), 217889_s_at | | CYBRD1 (0.486), 217889_s_at | ESR1 (0.353), 205225_at | | | | ESR1 (0.394), 205225_at |
| DCN (0.439), 209335_at | | ADRA2A (0.391), 209869_at | GJA1 (0.335), 201667_at | | | | RBBP8 (0.361), 203344_s_at |
| ADRA2A (0.416), 209869_at | | DCN (0.384), 209335_at | MGP (0.327), 202291_s_at | | | | PGR (0.347), 208305_at |
| SQLE (0.415), 209218_at | | SQLE (0.369), 209218_at | EPHX2 (0.313), 209368_at | | | | PTPRT (0.343), 205948_at |
| CXCL12 (0.388), 209687_at | | CCND1 (0.347), 208712_at | RBBP8 (0.311), 203344_s_at | | | | HSPA2 (0.317), 211538_s_at |
| EPHX2 (0.362), 209368_at | | ASPH (0.344), 210896_s_at | PTPRT (0.303), 205948_at | | | | PTGER3 (0.314), 210832_x_at |
| ASPH (0.352), 210896_s_at | | CXCL12 (0.342), 209687_at | PLAT (0.301), 201860_s_at | | | | |
| PRSS16 (0.352), 208165_s_at | | PIP (0.328), 206509_at | | | | | |

(continued)

| BIRC5 | RBBP8 | UBE2C | IL6ST | AZGP1 | DHCR7 | MGP | STC2 |
|---|---|---|---|---|---|---|---|
| EGFR (0.346), 201983_s_at | | PRSS16 (0.326), 208165_s_at | | | | | |
| CCND1 (0.331), 208712_at | | EGFR (0.320), 201983_s_at | | | | | |
| TRIM29 (0.325), 202504_at | | DHCR7 (0.315), 201791_s_at | | | | | |
| DHCR7 (0.323), 201791_s_at | | EPHX2 (0.315), 209368_at | | | | | |
| PIP (0.308), 206509_at | | TRIM29 (0.311), 202504_at | | | | | |
| TFAP2B (0.306), 214451_at | | | | | | | |
| WNT5A (0.303), 205990_s_at | | | | | | | |
| APOD (0.301), 201525_at | | | | | | | |
| PTPRT (0.301), 205948_at | | | | | | | |

The sequences of the primers and probes were as follows:

Table 1 Primer and probe sequences for the respective genes:

| **gene** | probe | **Seq ID** | **forward primer** | **Seq ID** | **reverse primer** | **Seq ID** |
|---|---|---|---|---|---|---|
| ABAT | TCGCCCTAAGAGGCTCT-TCCTC | 1 | GGCAACTTGAGGTCT-GACTTTTG | 2 | GGTCAGCTCACAAGTGGT-GTGA | 3 |
| ADRA2A | TTGTCCTTTCCCCCCTC-CGTGC | 4 | CCCCAAGAGCTGTTAGG-TATCAA | 5 | TCAATGACATGATCT-CAACCAGAA | 6 |
| APOD | CATCAGCTCTCAACTCCT-GGTTTAACA | 7 | ACTCACTAATGGAAAACG-GAAAGATC | 8 | TCACCTTCGATTTGAT-TCACAGTT | 9 |
| ASPH | TGGGAGGAAGGCAAGGT-GCTCATC | 10 | TGTGCCAACGAGACCAA-GAC | 11 | TCGTGCTCAAAGGAGT-CATCA | 12 |
| AURKA | CCGTCAGCCTGTGCTAG-GCAT | 13 | AATCTGGAGGCAAGGTTC-GA | 14 | TCTGGATTTGCCTCCTGT-GAA | 15 |
| BIRC5 | AGCCAGATGACGAC-CCCATAGAGGAACA | 16 | CCCAGTGTTTCTTCTGCT-TCAAG | 17 | CAACCGGACGAAT-GCTTTTT | 18 |
| CELSR2 | ACTGACTTTCCTTCT-GGAGCAGGTGGC | 19 | TCCAAGCATGTATTCCA-GACTTGT | 20 | TGCCCACAGCCTCTTTT-TCT | 21 |
| CHPT1 | CCACGGCCACCGAAGAG-GCAC | 22 | CGCTCGTGCTCATCTC-CTACT | 23 | CCCAGTGCACATAAAAGG-TATGTC | 24 |
| CXCL12 | CCACAGCAGGGTTTCAG-GTTCC | 25 | GCCACTACCCCCTCCT-GAA | 26 | TCACCTTGCCAACAGT-TCTGAT | 27 |
| CYBRD1 | AGGGCATCGCCAT-CATCGTC | 28 | GTCACCGGCTTCGTCTTCA | 29 | CAGGTCCACGGCAGTCT-GT | 30 |
| DCN | TCTTTTCAGCAACCCG-GTCCA | 31 | AAGGCTTCTTATTCGGGT-GTGA | 32 | TGGATGGCTGTATCTC-CCAGTA | 33 |
| DHCR7 | TGAGCGCCCACCCTCTC-GA | 34 | GGGCTCTGCTTCCCGATT | 35 | AGTCATAGGGCAAGCA-GAAAATTC | 36 |
| E2F8 | CAGGATACCTAATC-CCTCTCACGCAG | 37 | AAATGTCTCCGCAACCTT-GTTC | 38 | CTGCCCCCAGGGATGAG | 39 |
| EPHX2 | TGAAGCGGGAG-GACTTTTTGTAAA | 40 | CGATGAGAGTGTTTTATC-CATGCA | 41 | GCTGAGGCTGGGCTCT-TCT | 42 |
| ESR1 | ATGCCCTTTTGCCGATGCA | 43 | GCCAAATTGTGTTTGAT-GGATTAA | 44 | GACAAAACCGAGTCACAT-CAGTAATAG | 45 |
| GJA1 | TGCACAGCCTTTTGATTTC-CCCGAT | 46 | CGGGAAGCAC-CATCTCTAACTC | 47 | TTCATGTCCAG-CAGCTAGTTTTTT | 48 |
| HSPA2 | CAAGTCAGCAAACACG-CAAAA | 49 | CATGCACGAACTAAT-CAAAAATGC | 50 | ACATTATTCGAGGTT-TCTCTTTAATGC | 51 |

(continued)

| gene | probe | Seq ID | forward primer | Seq ID | reverse primer | Seq ID |
|---|---|---|---|---|---|---|
| IL6ST | CAAGCTCCACCTTC-CAAAGGACCT | 52 | CCCTGAATCCATAAAG-GCATACC | 53 | CAGCTTCGTTTTTC-CCTACTTTTT | 54 |
| INPP4B | TCCGAGCGCTGGATT-GCATGAG | 55 | GCACCAGTTACACAAG-GACTTCTTT | 56 | TCTCTATGCGGCATCCT-TCTC | 57 |
| MAPT | AGACTATTTGCACACT-GCCGCCT | 58 | GTGGCTCAAAGGA-TAATATCAAACAC | 59 | ACCTTGCTCAGGTCAACT-GGTT | 60 |
| MGP | CCTTCATATCCCCTCAG-CAGAGATGG | 61 | CCTTCATTAACAGGA-GAAATGCAA | 62 | ATTGAGCTCGTGGACAG-GCTTA | 63 |
| NEK2 | TCCTGAACAAATGAATCG-CATGTCCTACAA | 64 | ATTTGTTGGCACACCTTAT-TACATGT | 65 | AAGCAGCCCAATGACCA-GATa | 66 |
| PCNA | AAATACTAAAATGCGCCG-GCAATGA | 67 | GGGCGTGAACCTCAC-CAGTA | 68 | CTTCGGCCCTTAGTG-TAATGATATC | 69 |
| PGR | TTGATAGAAACGCTGT-GAGCTCGA | 70 | AGCTCATCAAGGCAATT-GGTTT | 71 | ACAAGATCATGCAAGT-TATCAAGAAGTT | 72 |
| PIP | TGCATGGTGGTTAAAACT-TACCTCA | 73 | TGCTTGCAGTTCAAACA-GAATTG | 74 | CACCTTGTAGAGGGAT-GCTGCTA | 75 |
| PLAT | CAGAAAGTGGCCAT-GCCACCCTG | 76 | TGGGAAGACATGAAT-GCACACTA | 77 | GGAGGTTGGGCTTTAGCT-GAA | 78 |
| PRSS16 | CACTGCCGGTCAC-CCACACCA | 79 | CTGAGGAGCACAGAACCT-CAACT | 80 | CGAACTCGGTACATGTCT-GATACAA | 81 |
| PTGER3 | TCGGTCTGCTGGTCTC-CGCTCC | 82 | CTGATTGAAGATCATTT-TCAACATCA | 83 | GACGGCCATTCAGCTTAT-GG | 84 |
| PTPRT | TTGGCTTCTGGACACCCT-CACA | 85 | GAGTTGTGGCCTCTAC-CATTGC | 86 | GAGCGGGAACCTTGGGA-TAG | 87 |
| RACGAP1 | ACTGAGAATCTCCACCCG-GCGCA | 88 | TCGCCAACTGGATAAATT-GGA | 89 | GAATGTGCGGAATCT-GTTTGAG | 90 |
| RBBP8 | ACCGATTCCGCTACATTC-CACCCAAC | 91 | AGAAATTGGCTTCCTGCT-CAAG | 92 | AAAACCAACTTC-CCAAAAATTCTCT | 93 |
| SCUBE2 | CTAGAGGGTTCCAGGTC-CCATACGTGACATA | 94 | TGTGGATTCAGT-TCAAGTCCAATG | 95 | CCATCTCGAACTATGTCT-TCAATGAGT | 96 |
| SEC14L2 | TGGGAGGCAT-GCAACGCGTG | 97 | AGGTCTTACTAAGCAGTC-CCATCTCT | 98 | CGACCGGCACCTGAACTC | 99 |
| SQLE | TATGCGTCTCCCAAAA-GAAGAACACCTCG | 100 | GCAAGCTTCCTTCCTCCT-TCA | 101 | CCTTTAGCAGTTTTCTC-CATAGTTTTATATC | 102 |

(continued)

| **gene** | probe | **Seq ID** | **forward primer** | **Seq ID** | **reverse primer** | **Seq ID** |
|---|---|---|---|---|---|---|
| TFAP2B | CAACACCACCACTAACAG-GCACACGTC | 103 | GGCATGGACAAGATGT-TCTTGA | 104 | CCTCCTTGTCGCCAGTTT-TACT | 105 |
| TOP2A | CAGATCAGGACCAAGAT-GGTTCCCACAT | 106 | CATTGAAGACGCTTCGT-TATGG | 107 | CCAGTTGTGATGGA-TAAAATTAATCAG | 108 |
| TRIM29 | TGCTGTCTCACTACCG-GCCATTCTACG | 109 | TGGAAATCTGGCAAGCA-GACT | 110 | CAATCCCGTTGCCTTT-GTTG | 111 |
| UBE2C | TGAACACACATGCT-GCCGAGCTCTG | 112 | CTTCTAGGAGAACCCAA-CATTGATAGT | 113 | GTTTCTTGCAGGTACTTCT-TAAAAGCT | 114 |
| WNT5A | TATTCACATCCCCTCAGTT-GCAGTGAATTG | 115 | CTGTGGCTCTTAATTTATT-GCATAATG | 116 | TTAGTGCTTTTTGCTT-TCAAGATCTT | 117 |
| STC2 | TCTCACCTTGACCCT-CAGCCAAG | 118 | ACATTTGACAAATTTCCCT-TAGGATT | 119 | CCAGGACGCAGCTTTAC-CAA | 120 |

A second alternative for unsupervised selection of possible gene replacement candidates is based on Affymetrix data only. This has the advantage that it can be done solely based on already published data (e.g. from www.ncbi.nlm.nih.gov/geo/). The following tables lists HG-U133a probe set replacement candidates for the probe sets used in algorithms T1 - T5. This is based on training data of these algorithms. The column header contains the gene name and the probe set ID in bold. Then, the 10 best-correlated probe sets are listed, where each table cell contains the probe set ID, the correlation coefficient in brackets and the gene name.

| UBE2C | BIRC5 | DHCR7 | RACGAP1 | AURKA | PVALB | NMU | STC2 |
|---|---|---|---|---|---|---|---|
| **202954_at** | **202095_s_at** | **201791_s_at** | **222077_s_at** | **204092_s_at** | **205336_at** | **206023_at** | **203438_at** |
| 210052_s_ at (0.82) TPX2 | 202954_at (0.82) UBE2C | 201790_s_at(0.66) DHCR7 | 218039_at (0.79) NUSAP1 | 208079_s_at(0.89) STK6 | 208683_at (-0.33) CAPN2 | 205347_s_at (0.45) TMSL8 | 203439_s_at (0.88) STC2 |
| 202095_s_ at (0.82) BIRC5 | 218039_at (0.81) NUSAP1 | 202218_s_at(0.48) FADS2 | 214710_s_at(0.78) CCNB1 | 202954_at (0.80) UBE2C | 219682_s_at (0.30) TBX3 | 203764_at (0.45) DLG7 | 212496_s_at (0.52) JMJD2B |
| 218009_s_ at (0.82) PRC1 | 218009_s_at(0.79) PRC1 | 202580_x_at(0.47) FOXM1 | 203764_at (0.77) DLG7 | 210052_s_at(0.77) TPX2 | 218704_at (0.30) FLJ20315 | 203554_x_at (0.44) PTTG1 | 219440_at (0.52) RAI2 |
| 203554_x_ at (0.82) PTTG1 | 202705_at (0.78) CCNB2 | 208944_at (-0.46) TGFBR2 | 204026_s_at(0.77) ZWINT | 202095_s_at(0.77) BIRC5 | | 204962_s_at (0.44) CENPA | 215867_x_at (0.51) CA12 |
| 208079_s_ at (0.81) STK6 | 204962_s_at(0.78) CENPA | 202954_at (0.46) UBE2C | 218009_s_at(0.76) PRC1 | 203554_x_at(0.76) PTTG1 | | 204825_at (0.43) MELK | 214164_x_at (0.50) CA12 |
| 202705_at (0.81) CCNB2 | 203554_x_at(0.78) PTTG1 | 209541_at (-0.45) IGF1 | 204641_at (0.76) NEK2 | 218009_s_at(0.75) PRC1 | | 209714_s_at (0.41) CDKN3 | 204541_at (0.50) SEC14L2 |
| 218039_at (0.81) NUSAP1 | 208079_s_at(0.78) STK6 | 201059_at (0.45) CTTN | 204444_at (0.75) KIF11 | 201292_at (0.73) TOP2A | | 219918_s_at (0.41) ASPM | 203963_at (0.50) CA12 |
| 202870_s_ at (0.80) CDC20 | 210052_s_at(0.77) TPX2 | 200795_at (-0.45) SPARCL1 | 202705_at (0.75) CCNB2 | 214710_s_at(0.73) CCNB1 | | 207828_s_at (0.41) CENPF | 212495_at (0.50) JMJD2B |
| 204092_s_ at (0.80) STK6 | 202580_x_at(0.77) FOXM1 | 218009_s_at(0.45) PRC1 | 203362_s_at(0.75) MAD2L1 | 204962_s_at(0.73) CENPA | | 202705_at (0.41) CCNB2 | 208614_s_at (0.49) FLNB |
| 209408_at (0.80) KIF2C | 204092_s_at(0.77) STK6 | 218542_at (0.45) C10orf3 | 202954_at (0.75) UBE2C | 218039_at (0.73) NUSAP1 | | 219787_s_at (0.40) ECT2 | 213933_at (0.49) PTGER3 |

| **AZGP1** | **RBBP8** | **IL6ST** | **MGP** | **PTGER3** | **CXCL12** | **ABAT** | **CDH1** |
|---|---|---|---|---|---|---|---|
| **209309_at** | **203344_s_at** | **212196_at** | **202291_s_at** | **213933_at** | **209687_at** | **209460_at** | **201131_s_at** |
| 217014_s_at (0.92) AZGP1 | 36499_at (0.49) CELSR2 | 212195_at (0.85) IL6ST | 201288_at (0.46) ARHGDIB | 210375_at (0.74) PTGER3 | 204955_at (0.81) SRPX | 209459_s_at (0.92) ABAT | 201130_s_at (0.57) CDH1 |
| 206509_at (0.52) PIP | 204029_at (0.45) CELSR2 | 204864_s_at (0.75) IL6ST | 219768_at (0.42) VTCN1 | 210831_s_at (0.74) PTGER3 | 209335_at (0.81) DCN | 206527_at (0.63) ABAT | 221597_s_at (0.40) HSPC171 |
| 204541_at (0.46) SEC14L2 | 208305_at (0.45) PGR | 211000_s_at (0.68) IL6ST | 202849_x_at (-0.41) GRK6 | 210374_x_at (0.73) PTGER3 | 211896_s_at (0.81) DCN | 213392_at (0.54) MGC35048 | 203350_at (0.38) AP1G1 |
| 200670_at (0.45) XBP1 | 205380_at (0.43) PDZK1 | 214077_x_at (0.61) MEIS4 | 205382_s_at (0.40) DF | 210832_x_at (0.73) PTGER3 | 201893_x_at (0.81) DCN | 221666_s_at (0.49) PYCARD | 209163_at (0.36) CYB561 |
| 209368_at (0.45) EPHX2 | 203303_at (0.41) TCTE1L | 204863_s_at (0.58) IL6ST | 200099_s_at (0.39) RPS3A | 210834_s_at (0.55) PTGER3 | 203666_at (0.80) CXCL12 | 218016_s_at (0.48) POLR3E | 210239_at (0.35) IRX5 |
| 218627_at (-0.43) FLJ11259 | 205280_at (0.38) GLRB | 202089_s_at (0.57) SLC39A6 | 221591_s_at (-0.37) FAM64A | 210833_at (0.55) PTGER3 | 211813_x_at (0.80) DCN | 214440_at (0.46) NAT1 | 200942_s_at (0.34) HSBP1 |
| 202286_s_at (0.43) TACSTD2 | 205279_s_at (0.38) GLRB | 210735_s_at (0.56) CA12 | 214629_x_at (0.37) RTN4 | 203438_at (0.49) STC2 | 208747_s_at (0.79) C1S | 204981_at (0.45) SLC22A18 | 209157_at (0.34) DNAJA2 |
| 213832_at (0.42) --- | 203685_at (0.38) BCL2 | 200648_s_at (0.52) GLUL | 200748_s_at (0.37) FTH1 | 203439_s_at (0.46) STC2 | 203131_at (0.78) PDGFRA | 212195_at (0.45) IL6ST | 210715_s_at (0.33) SPINT2 |
| 204288_s_at (0.41) SORBS2 | 203304_at (-0.38) BAMBI | 214552_s_at (0.52) RABEP1 | 209408_at (-0.37) KIF2C | 212195_at (0.41) IL6ST | 202994_s_at (0.78) FBLN1 | 204497_at (0.45) ADCY9 | 203219_s_at (0.33) APRT |
| 202376_at (0.41) SERPINA3 | 205862_at (0.36) GREB1 | 219197_s_at (0.51) SCUBE2 | 218726_at (-0.36) DKFZp762E1312 | 217764_s_at (0.40) RAB31 | 208944_at (0.78) TGFBR2 | 215867_x_at (0.45) CA12 | 218074_at (0.33) FAM96B |

After selection of a gene or a probe set one has to define a mathematical mapping between the expression values of the gene to replace and those of the new gene. There are several alternatives which are discussed here based on the example "replace delta-Ct values of BIRC5 by RACGAP1". In the training data the joint distribution of expressions looks like this:

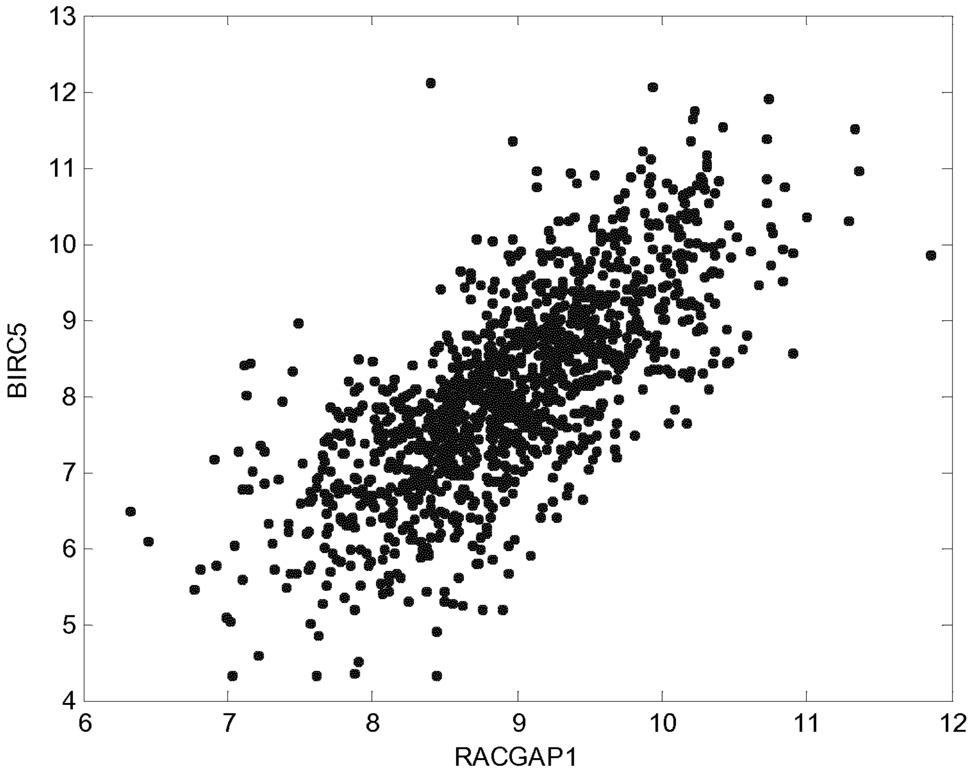


The Pearson correlation coefficient is 0.73.

One approach is to create a mapping function from RACGAP1 to BIRC5 by regression. Linear regression is the first choice and yields in this example

$$BIRC5 = 1.22 * RACGAP1 - 2.85.$$

Using this equation one can easily replace the BIRC5 variable in e.g. algorithm T5 by the right hand side. In other examples robust regression, polynomial regression or univariate nonlinear pre-transformations may be adequate.

The regression method assumes measurement noise on BIRC5, but no noise on RACGAP1. Therefore the mapping is not symmetric with respect to exchangeability of the two variables. A symmetric mapping approach would be based on two univariate z-transformations.

$$z = (BIRC5 - mean(BIRC5)) / std(BIRC5)$$

and

$$z = (RACGAP1 - mean(RACGAP1)) / std(RACGAP1)$$

$$z = (BIRC5 - 8.09) / 1.29 = (RACGAP1 - 8.95) / 0.77$$

$$BIRC5 = 1.67 * RACGAP1 + -6.89$$

Again, in other examples, other transformations may be adequate: normalization by median and/or mad, nonlinear mappings, or others.

**Claims**

1. A method for predicting a response to and/or benefit of taxane/anthracycline-containing chemotherapy, including neoadjuvant chemotherapy, in a patient suffering from or at risk of developing recurrent breast cancer, said method comprising the steps of:

   (a) determining in a breast tumor sample from said patient the gene expression levels of at least 3 of the following genes: BIRC5, DHCR7, AZGP1, RBBP8, IL6ST, and MGP, indicative of a response to chemotherapy for a tumor, and
   (b) mathematically combining expression level values for the genes of the said set which values were determined in the tumor sample to yield a combined score, wherein said combined score is predicting said response and/or benefit of chemotherapy.

2. The method of claim 1 for predicting a response to cytotoxic chemotherapy, preferably in Her2/neu negative, estrogen receptor positive (luminal) tumors, preferably in the neoadjuvant mode.

3. The method of any one of the foregoing claims, wherein said expression level is determined as a non-protein such as a gene expression level.

4. The method of any one of the foregoing claims, wherein said expression level is determined by at least one of

a PCR based method,
a micorarray based method, or
a hybridization based method, a sequencing and/or next generation sequencing approach.

5. The method of any one of the foregoing claims, wherein said determination of expression levels is in a formalin-fixed paraffin-embedded tumor sample or in a fresh-frozen tumor sample.

6. The method of any one of the foregoing claims, wherein the expression level of said at least one marker gene is determined as a pattern of expression relative to at least one reference gene or to a computed average expression value.

7. The method of any one of the foregoing claims, wherein said step of mathematically combining comprises a step of applying an algorithm to values representative of an expression level of a given gene, in particular wherein said algorithm is a linear combination of said values representative of an expression level of a given gene, or wherein a value for a representative of an expression level of a given gene is multiplied with a coefficient.

8. The method of any one of the foregoing claims, wherein one, two or more thresholds are determined for said combined score and discriminated into high and low risk, high, intermediate and low risk, or more risk groups by applying the threshold on the combined score.

9. The method of any one of the foregoing claims, wherein a high combined score is indicative of benefit from a more aggressive therapy, e.g. cytotoxic chemotherapy.

10. The method of any one of the foregoing claims, wherein information regarding nodal status of the patient is processed in the step of mathematically combining expression level values for the genes to yield a combined score.

11. The method of any one of the foregoing claims, wherein said information regarding tumor size of the patient is processed in the step of mathematically combining expression level values for the genes to yield a combined score.

12. The method of any one of the foregoing claims, wherein said information regarding nodal status and tumor size of the patient is processed in the step of mathematically combining expression level values for the genes to yield a combined score.

## Patentansprüche

1. Verfahren zur Vorhersage eines Ansprechens auf und/oder Nutzens einer Chemotherapie mit Taxan/Anthracyclin einschließlich einer Neoadjuvans-Chemotherapie bei einem Patienten, der an rezidivierendem Brustkrebs leidet oder bei dem das Risiko besteht, dass er einen solchen entwickelt, wobei das Verfahren die folgenden Schritte umfasst:

(a) Bestimmen der Gen-Expressionsniveaus von wenigstens 3 der folgenden Gene in einer Brusttumorprobe von dem Patienten: BIRC5, DHCR7, AZGP1, RBBP8, IL6ST und MGP, die ein Ansprechen auf Chemotherapie für einen Tumor anzeigen; und
(b) mathematisches Kombinieren der Expressionsniveauwerte für die Gene der genannten Menge, wobei die Werte in der Tumorprobe bestimmt wurden, unter Erhalt eines kombinierten Score, wobei der kombinierte Score das Ansprechen auf und/oder den Nutzen einer Chemotherapie vorhersagt.

2. Verfahren gemäß Anspruch 1 zur Vorhersage eines Ansprechens auf cytotoxische Chemotherapie, vorzugsweise bei Her2/neu-negativen, Östrogenrezeptor-positiven (luminalen) Tumoren, vorzugsweise im Neoadjuvans-Modus.

3. Verfahren gemäß einem der vorstehenden Ansprüche, wobei das Expressionsniveau als Nichtprotein, wie auf dem Genexpressionsniveau, bestimmt wird.

4. Verfahren gemäß einem der vorstehenden Ansprüche, wobei das Expressionsniveau durch wenigstens eines der folgenden Verfahren bestimmt wird:

   ein Verfahren auf PCR-Basis;
   ein Verfahren auf Mikroarray-Basis; oder
   ein Verfahren auf Hybridisierungsbasis, einen Sequenzierungs- und/oder Nächste-Generation-Sequenzierungsansatz.

5. Verfahren gemäß einem der vorstehenden Ansprüche, wobei die Bestimmung der Expressionsniveaus in einer mit Formalin fixierten und in Paraffin eingebetteten Tumorprobe oder in einer frisch eingefrorenen Tumorprobe erfolgt.

6. Verfahren gemäß einem der vorstehenden Ansprüche, wobei das Expressionsniveau des wenigstens einen Marker-Gens als Expressionsmuster relativ zu wenigstens einem Referenz-Gen oder zu einem berechneten mittleren Expressionswert bestimmt wird.

7. Verfahren gemäß einem der vorstehenden Ansprüche, wobei der Schritt des mathematischen Kombinierens einen Schritt des Anwendens eines Algorithmus auf Werte, die repräsentativ für ein Expressionsniveau eines gegebenen Gens sind, umfasst, wobei der Algorithmus insbesondere eine Linearkombination der Werte ist, die repräsentativ für ein Expressionsniveau eines gegebenen Gens sind, oder wobei ein Wert, der repräsentativ für ein Expressionsniveau eines gegebenen Gens ist, mit einem Koeffizienten multipliziert wird.

8. Verfahren gemäß einem der vorstehenden Ansprüche, wobei eine, zwei oder mehr Schwellen für den kombinierten Score bestimmt werden, die zwischen hohen und geringem Risiko, hohem, mittlerem und geringem Risiko oder mehr Risikogruppen diskriminieren, indem man die Schwelle auf den kombinierten Score anwendet.

9. Verfahren gemäß einem der vorstehenden Ansprüche, wobei ein hoher kombinierter Score den Nutzen einer aggressiveren Therapie, zum Beispiel einer cytotoxischen Chemotherapie, anzeigt.

10. Verfahren gemäß einem der vorstehenden Ansprüche, wobei in dem Schritt des mathematischen Kombinierens der Expressionsniveauwerte für die Gene Informationen bezüglich des Nodalstatus des Patienten verarbeitet werden, wobei man einen kombinierten Score erhält.

11. Verfahren gemäß einem der vorstehenden Ansprüche, wobei in dem Schritt des mathematischen Kombinierens der Expressionsniveauwerte für die Gene Informationen bezüglich der Tumorgröße des Patienten verarbeitet werden, wobei man einen kombinierten Score erhält.

12. Verfahren gemäß einem der vorstehenden Ansprüche, wobei in dem Schritt des mathematischen Kombinierens der Expressionsniveauwerte für die Gene Informationen bezüglich des Nodalstatus und der Tumorgröße des Patienten verarbeitet werden, wobei man einen kombinierten Score erhält.

## Revendications

1. Procédé de prédiction d'une réponse à et/ou d'un bénéfice d'une chimiothérapie contenant du taxane/de l'anthracycline, y compris une chimiothérapie néoadjuvante, chez un patient souffrant de ou présentant un risque de développer un cancer du sein récurrent, ledit procédé comprenant les étapes de :

   (a) détermination dans un échantillon de tumeur du sein auprès dudit patient des niveaux d'expression de gène d'au moins 3 des gènes suivants : BIRC5, DHCR7, AZGP1, RBBP8, IL6ST, et MGP, indicatifs d'une réponse à la chimiothérapie concernant une tumeur, et
   (b) combinaison mathématique des valeurs de niveau d'expression pour les gènes dudit jeu, lesquelles valeurs ont été déterminées dans l'échantillon de tumeur pour donner un score combiné, dans lequel ledit score combiné est prédictif de ladite réponse et/ou dudit bénéfice de la chimiothérapie.

2. Procédé selon la revendication 1, de prédiction d'une réponse à une chimiothérapie cytotoxique, de préférence dans des tumeurs (luminales) négatives à Her2/neu, positives au récepteur oestrogénique, de préférence dans le mode néoadjuvant.

3. Procédé selon l'une quelconque des revendications précédentes, dans lequel ledit niveau d'expression est déterminé sous forme d'une non-protéine tel qu'un niveau d'expression de gène.

4. Procédé selon l'une quelconque des revendications précédentes, dans lequel ledit niveau d'expression est déterminé par au moins l'un parmi

un procédé à base de PCR,
un procédé à base de puces à ADN, ou
un procédé à base d'hybridation, un séquençage et/ou une approche de séquençage de nouvelle génération.

5. Procédé selon l'une quelconque des revendications précédentes, dans lequel ladite détermination de niveaux d'expression est réalisée dans un échantillon de tumeur fixé par formaline imprégné de paraffine ou dans un échantillon de tumeur frais congelé.

6. Procédé selon l'une quelconque des revendications précédentes, dans lequel le niveau d'expression dudit au moins un gène marqueur est déterminé comme un modèle d'expression relatif à au moins un gène de référence ou à une valeur d'expression moyenne calculée.

7. Procédé selon l'une quelconque des revendications précédentes, dans lequel ladite étape de combinaison mathématique comprend une étape d'application d'un algorithme à des valeurs représentatives d'un niveau d'expression d'un gène donné, en particulier dans lequel ledit algorithme est une combinaison linéaire desdites valeurs représentatives d'un niveau d'expression d'un gène donné, ou dans lequel une valeur représentative d'un niveau d'expression d'un gène donné est multipliée par un coefficient.

8. Procédé selon l'une quelconque des revendications précédentes, dans lequel un, deux ou plusieurs seuils sont déterminés pour ledit score combiné et discriminés en groupes à risque élevé et faible, élevé, intermédiaire et faible, ou d'autres groupes de risque en appliquant le seuil au score combiné.

9. Procédé selon l'une quelconque des revendications précédentes, dans lequel un score combiné élevé est indicatif d'un bénéfice tiré d'une thérapie plus agressive, par exemple une chimiothérapie cytotoxique.

10. Procédé selon l'une quelconque des revendications précédentes, dans lequel des informations concernant le statut nodal du patient sont traitées dans l'étape de combinaison mathématique de valeurs de niveau d'expression pour les gènes pour donner un score combiné.

11. Procédé selon l'une quelconque des revendications précédentes, dans lequel lesdites informations concernant une taille de tumeur du patient sont traitées dans l'étape de combinaison mathématique de valeurs de niveau d'expression pour les gènes pour donner un score combiné.

12. Procédé selon l'une quelconque des revendications précédentes, dans lequel lesdites informations concernant le statut nodal et la taille de tumeur du patient sont traitées dans l'étape de combinaison mathématique de valeurs de niveau d'expression pour les gènes pour donner un score combiné.

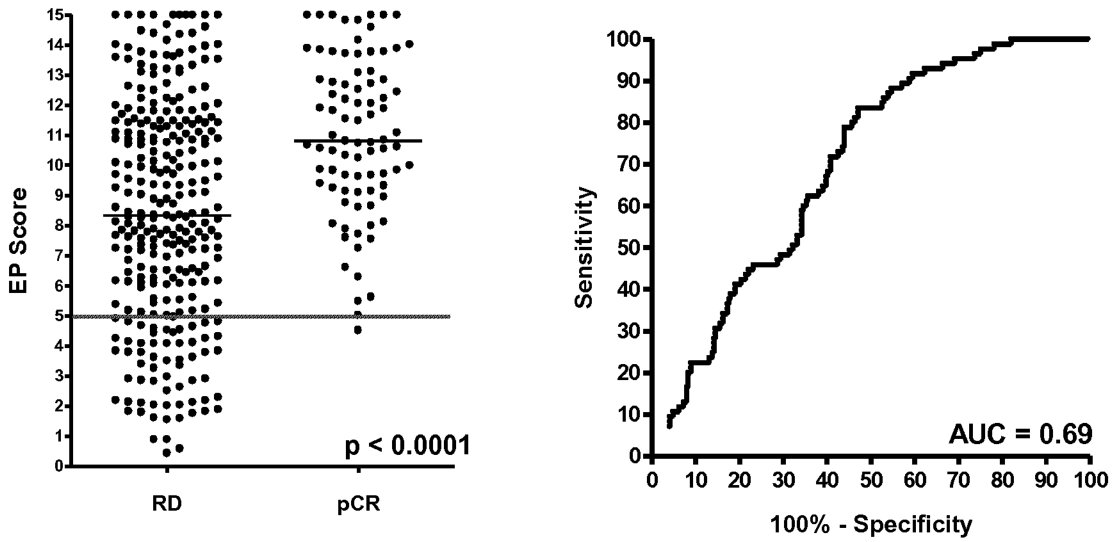


Figure 1

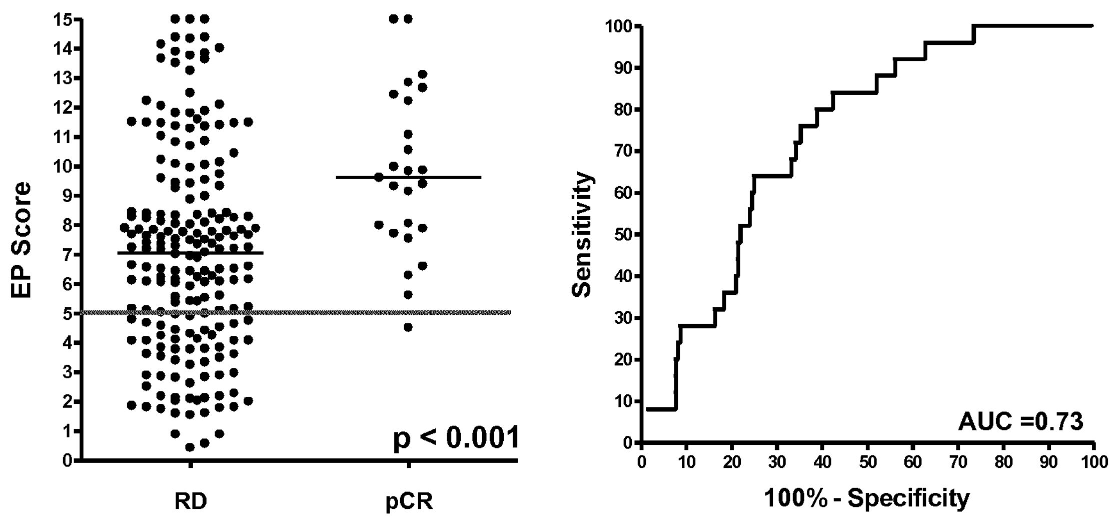


Figure 2

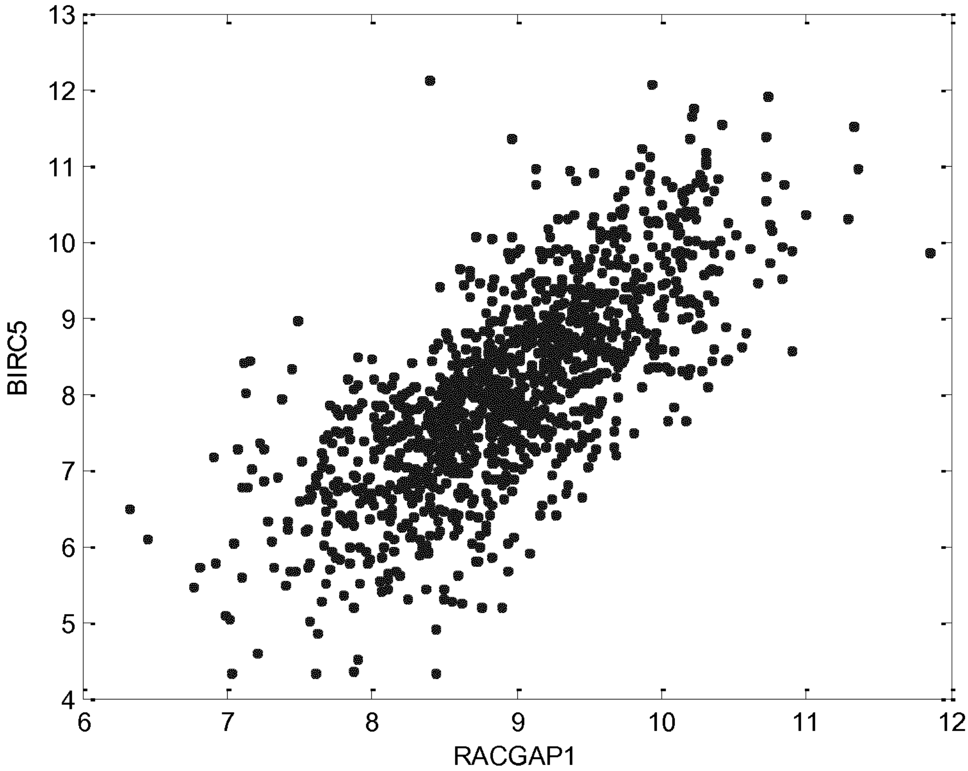


Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010076322 A1
- WO 2009158143 A1
- WO 2006119593 A
- WO 2008006517 A2
- WO 2009114836 A1
- WO 2011120984 A1
- EP 2012064865 W
- EP 11175852 A

### Non-patent literature cited in the description


- **JEMAL et al.** *CA Cancer J Clin.,* 2011
- **MISSET et al.** *J Clin Oncol.,* 1996
- **HENDERSON et al.** *J Clin Oncol.,* 2003
- **HESS et al.** *J Clin Oncol.,* 2006
- **TABCHY et al.** *Clin Can Res,* 2010
- **SOTIRIOU et al.** *JNCI,* 2006
- **LIEDTKE.** *J Clin Oncol,* 2009
- **MAIA CHANRION et al.** *Clin Cancer Res 2008,* 15 March 2008, vol. 14 (6), 1744-1752
- **KAREN J TAYLOR et al.** *Breast Cancer Research,* 2010, vol. 12, R39

SEQUENCE LISTING

<110> Sividon Diagnostics GmbH

<120> Method for predicting the response to chemotherapy in a patient suffering from or at risk of developing recurrent breast cancer

<130> 161573EP

<140> PCT/EP2012/064865
<141> 2012-07-30

<150> EP 11175852
<151> 2011-07-28

<160> 137

<170> PatentIn version 3.3

<210> 1
<211> 22
<212> DNA
<213> Homo sapiens

<400> 1
tcgccctaag aggctcttcc tc 22

<210> 2
<211> 23
<212> DNA
<213> Homo sapiens

<400> 2
ggcaacttga ggtctgactt ttg 23

<210> 3
<211> 22
<212> DNA
<213> Homo sapiens

<400> 3
ggtcagctca caagtggtgt ga 22

<210> 4
<211> 22
<212> DNA
<213> Homo sapiens

<400> 4
ttgtcctttc ccccctccgt gc 22

<210> 5
<211> 23
<212> DNA

<213> Homo sapiens

<400> 5
ccccaagagc tgttaggtat caa 23

<210> 6
<211> 24
<212> DNA
<213> Homo sapiens

<400> 6
tcaatgacat gatctcaacc agaa 24

<210> 7
<211> 27
<212> DNA
<213> Homo sapiens

<400> 7
catcagctct caactcctgg tttaaca 27

<210> 8
<211> 26
<212> DNA
<213> Homo sapiens

<400> 8
actcactaat ggaaaacgga aagatc 26

<210> 9
<211> 24
<212> DNA
<213> Homo sapiens

<400> 9
tcaccttcga tttgattcac agtt 24

<210> 10
<211> 24
<212> DNA
<213> Homo sapiens

<400> 10
tgggaggaag gcaaggtgct catc 24

<210> 11
<211> 20
<212> DNA
<213> Homo sapiens

<400> 11
tgtgccaacg agaccaagac 20

<210> 12
<211> 21
<212> DNA
<213> Homo sapiens

<400> 12
tcgtgctcaa aggagtcatc a 21

<210> 13
<211> 21
<212> DNA
<213> Homo sapiens

<400> 13
ccgtcagcct gtgctaggca t 21

<210> 14
<211> 20
<212> DNA
<213> Homo sapiens

<400> 14
aatctggagg caaggttcga 20

<210> 15
<211> 21
<212> DNA
<213> Homo sapiens

<400> 15
tctggatttg cctcctgtga a 21

<210> 16
<211> 28
<212> DNA
<213> Homo sapiens

<400> 16
agccagatga cgaccccata gaggaaca 28

<210> 17
<211> 23
<212> DNA
<213> Homo sapiens

<400> 17
cccagtgttt cttctgcttc aag 23

<210> 18
<211> 20
<212> DNA
<213> Homo sapiens

<400> 18
caaccggacg aatgcttttt 20

<210> 19
<211> 27
<212> DNA
<213> Homo sapiens

<400> 19
actgactttc cttctggagc aggtggc 27

<210> 20
<211> 24
<212> DNA
<213> Homo sapiens

<400> 20
tccaagcatg tattccagac ttgt 24

<210> 21
<211> 20
<212> DNA
<213> Homo sapiens

<400> 21
tgcccacagc ctctttttct 20

<210> 22
<211> 21
<212> DNA
<213> Homo sapiens

<400> 22
ccacggccac cgaagaggca c 21

<210> 23
<211> 21
<212> DNA
<213> Homo sapiens

<400> 23
cgctcgtgct catctcctac t 21

<210> 24
<211> 24
<212> DNA
<213> Homo sapiens

<400> 24
cccagtgcac ataaaaggta tgtc 24

<210> 25
<211> 22
<212> DNA
<213> Homo sapiens

<400> 25
ccacagcagg gtttcaggtt cc 22

<210> 26
<211> 19
<212> DNA
<213> Homo sapiens

<400> 26
gccactaccc cctcctgaa 19

<210> 27
<211> 22

<212> DNA
<213> Homo sapiens

<400> 27
tcaccttgcc aacagttctg at 22

<210> 28
<211> 20
<212> DNA
<213> Homo sapiens

<400> 28
agggcatcgc catcatcgtc 20

<210> 29
<211> 19
<212> DNA
<213> Homo sapiens

<400> 29
gtcaccggct tcgtcttca 19

<210> 30
<211> 19
<212> DNA
<213> Homo sapiens

<400> 30
caggtccacg gcagtctgt 19

<210> 31
<211> 21
<212> DNA
<213> Homo sapiens

<400> 31
tcttttcagc aacccggtcc a 21

<210> 32
<211> 22
<212> DNA
<213> Homo sapiens

<400> 32
aaggcttctt attcgggtgt ga 22

<210> 33
<211> 22
<212> DNA
<213> Homo sapiens

<400> 33
tggatggctg tatctcccag ta 22

<210> 34
<211> 19
<212> DNA
<213> Homo sapiens

<400> 34
tgagcgccca ccctctcga 19

<210> 35
<211> 18
<212> DNA
<213> Homo sapiens

<400> 35
gggctctgct tcccgatt 18

<210> 36
<211> 24
<212> DNA
<213> Homo sapiens

<400> 36
agtcataggg caagcagaaa attc 24

<210> 37
<211> 26
<212> DNA
<213> Homo sapiens

<400> 37
caggatacct aatccctctc acgcag 26

<210> 38
<211> 22
<212> DNA
<213> Homo sapiens

<400> 38
aaatgtctcc gcaaccttgt tc 22

<210> 39
<211> 17
<212> DNA
<213> Homo sapiens

<400> 39
ctgcccccag ggatgag 17

<210> 40
<211> 24
<212> DNA
<213> Homo sapiens

<400> 40
tgaagcggga ggactttttg taaa 24

<210> 41
<211> 24
<212> DNA
<213> Homo sapiens

<400> 41
cgatgagagt gttttatcca tgca 24

<210> 42
<211> 19
<212> DNA
<213> Homo sapiens

<400> 42
gctgaggctg ggctcttct 19

<210> 43
<211> 19
<212> DNA
<213> Homo sapiens

<400> 43
atgccctttt gccgatgca 19

<210> 44
<211> 24
<212> DNA
<213> Homo sapiens

<400> 44
gccaaattgt gtttgatgga ttaa 24

<210> 45
<211> 27
<212> DNA
<213> Homo sapiens

<400> 45
gacaaaaccg agtcacatca gtaatag 27

<210> 46
<211> 25
<212> DNA
<213> Homo sapiens

<400> 46
tgcacagcct tttgatttcc ccgat 25

<210> 47
<211> 22
<212> DNA
<213> Homo sapiens

<400> 47
cgggaagcac catctctaac tc 22

<210> 48
<211> 24
<212> DNA
<213> Homo sapiens

<400> 48
ttcatgtcca gcagctagtt tttt 24

<210> 49
<211> 21

<212> DNA
<213> Homo sapiens

<400> 49
caagtcagca aacacgcaaa a 21

<210> 50
<211> 24
<212> DNA
<213> Homo sapiens

<400> 50
catgcacgaa ctaatcaaaa atgc 24

<210> 51
<211> 27
<212> DNA
<213> Homo sapiens

<400> 51
acattattcg aggtttctct ttaatgc 27

<210> 52
<211> 24
<212> DNA
<213> Homo sapiens

<400> 52
caagctccac cttccaaagg acct 24

<210> 53
<211> 23
<212> DNA
<213> Homo sapiens

<400> 53
ccctgaatcc ataaaggcat acc 23

<210> 54
<211> 24
<212> DNA
<213> Homo sapiens

<400> 54
cagcttcgtt tttccctact tttt 24

<210> 55
<211> 22
<212> DNA
<213> Homo sapiens

<400> 55
tccgagcgct ggattgcatg ag 22

<210> 56
<211> 25
<212> DNA
<213> Homo sapiens

<400> 56
gcaccagtta cacaaggact tcttt 25

<210> 57
<211> 21
<212> DNA
<213> Homo sapiens

<400> 57
tctctatgcg gcatccttct c 21

<210> 58
<211> 23
<212> DNA
<213> Homo sapiens

<400> 58
agactatttg cacactgccg cct 23

<210> 59
<211> 26
<212> DNA
<213> Homo sapiens

<400> 59
gtggctcaaa ggataatatc aaacac 26

<210> 60
<211> 22
<212> DNA
<213> Homo sapiens

<400> 60
accttgctca ggtcaactgg tt 22

<210> 61
<211> 26
<212> DNA
<213> Homo sapiens

<400> 61
ccttcatatc ccctcagcag agatgg 26

<210> 62
<211> 24
<212> DNA
<213> Homo sapiens

<400> 62
ccttcattaa caggagaaat gcaa 24

<210> 63
<211> 22
<212> DNA
<213> Homo sapiens

<400> 63
attgagctcg tggacaggct ta 22

<210> 64
<211> 30
<212> DNA
<213> Homo sapiens

<400> 64
tcctgaacaa atgaatcgca tgtcctacaa 30

<210> 65
<211> 26
<212> DNA
<213> Homo sapiens

<400> 65
atttgttggc acaccttatt acatgt 26

<210> 66
<211> 21
<212> DNA
<213> Homo sapiens

<400> 66
aagcagccca atgaccagat a 21

<210> 67
<211> 25
<212> DNA
<213> Homo sapiens

<400> 67
aaatactaaa atgcgccggc aatga 25

<210> 68
<211> 20
<212> DNA
<213> Homo sapiens

<400> 68
gggcgtgaac ctcaccagta 20

<210> 69
<211> 25
<212> DNA
<213> Homo sapiens

<400> 69
cttcggccct tagtgtaatg atatc 25

<210> 70
<211> 24
<212> DNA
<213> Homo sapiens

<400> 70
ttgatagaaa cgctgtgagc tcga 24

<210> 71
<211> 22

<212> DNA
<213> Homo sapiens

<400> 71
agctcatcaa ggcaattggt tt 22

<210> 72
<211> 28
<212> DNA
<213> Homo sapiens

<400> 72
acaagatcat gcaagttatc aagaagtt 28

<210> 73
<211> 25
<212> DNA
<213> Homo sapiens

<400> 73
tgcatggtgg ttaaaactta cctca 25

<210> 74
<211> 23
<212> DNA
<213> Homo sapiens

<400> 74
tgcttgcagt tcaaacagaa ttg 23

<210> 75
<211> 23
<212> DNA
<213> Homo sapiens

<400> 75
caccttgtag agggatgctg cta 23

<210> 76
<211> 23
<212> DNA
<213> Homo sapiens

<400> 76
cagaaagtgg ccatgccacc ctg 23

<210> 77
<211> 23
<212> DNA
<213> Homo sapiens

<400> 77
tgggaagaca tgaatgcaca cta 23

<210> 78
<211> 21
<212> DNA
<213> Homo sapiens

<400> 78
ggaggttggg ctttagctga a 21

<210> 79
<211> 21
<212> DNA
<213> Homo sapiens

<400> 79
cactgccggt cacccacacc a 21

<210> 80
<211> 23
<212> DNA
<213> Homo sapiens

<400> 80
ctgaggagca cagaacctca act 23

<210> 81
<211> 25
<212> DNA
<213> Homo sapiens

<400> 81
cgaactcggt acatgtctga tacaa 25

<210> 82
<211> 22
<212> DNA
<213> Homo sapiens

<400> 82
tcggtctgct ggtctccgct cc 22

<210> 83
<211> 26
<212> DNA
<213> Homo sapiens

<400> 83
ctgattgaag atcattttca acatca 26

<210> 84
<211> 20
<212> DNA
<213> Homo sapiens

<400> 84
gacggccatt cagcttatgg 20

<210> 85
<211> 22
<212> DNA
<213> Homo sapiens

<400> 85
ttggcttctg gacaccctca ca 22

<210> 86
<211> 22
<212> DNA
<213> Homo sapiens

<400> 86
gagttgtggc ctctaccatt gc 22

<210> 87
<211> 20
<212> DNA
<213> Homo sapiens

<400> 87
gagcgggaac cttgggatag 20

<210> 88
<211> 23
<212> DNA
<213> Homo sapiens

<400> 88
actgagaatc tccacccggc gca 23

<210> 89
<211> 21
<212> DNA
<213> Homo sapiens

<400> 89
tcgccaactg gataaattgg a 21

<210> 90
<211> 22
<212> DNA
<213> Homo sapiens

<400> 90
gaatgtgcgg aatctgtttg ag 22

<210> 91
<211> 26
<212> DNA
<213> Homo sapiens

<400> 91
accgattccg ctacattcca cccaac 26

<210> 92
<211> 22
<212> DNA
<213> Homo sapiens

<400> 92
agaaattggc ttcctgctca ag 22

<210> 93
<211> 25

<212> DNA
<213> Homo sapiens

<400> 93
aaaaccaact tcccaaaaat tctct 25

<210> 94
<211> 31
<212> DNA
<213> Homo sapiens

<400> 94
ctagagggtt ccaggtccca tacgtgacat a 31

<210> 95
<211> 24
<212> DNA
<213> Homo sapiens

<400> 95
tgtggattca gttcaagtcc aatg 24

<210> 96
<211> 27
<212> DNA
<213> Homo sapiens

<400> 96
ccatctcgaa ctatgtcttc aatgagt 27

<210> 97
<211> 20
<212> DNA
<213> Homo sapiens

<400> 97
tgggaggcat gcaacgcgtg 20

<210> 98
<211> 26
<212> DNA
<213> Homo sapiens

<400> 98
aggtcttact aagcagtccc atctct 26

<210> 99
<211> 18
<212> DNA
<213> Homo sapiens

<400> 99
cgaccggcac ctgaactc 18

<210> 100
<211> 29
<212> DNA
<213> Homo sapiens

<400> 100
tatgcgtctc ccaaaagaag aacacctcg 29

<210> 101
<211> 21
<212> DNA
<213> Homo sapiens

<400> 101
gcaagcttcc ttcctccttc a 21

<210> 102
<211> 31
<212> DNA
<213> Homo sapiens

<400> 102
cctttagcag ttttctccat agttttatat c 31

<210> 103
<211> 27
<212> DNA
<213> Homo sapiens

<400> 103
caacaccacc actaacaggc acacgtc 27

<210> 104
<211> 22
<212> DNA
<213> Homo sapiens

<400> 104
ggcatggaca agatgttctt ga 22

<210> 105
<211> 22
<212> DNA
<213> Homo sapiens

<400> 105
cctccttgtc gccagtttta ct 22

<210> 106
<211> 28
<212> DNA
<213> Homo sapiens

<400> 106
cagatcagga ccaagatggt tcccacat 28

<210> 107
<211> 22
<212> DNA
<213> Homo sapiens

<400> 107
cattgaagac gcttcgttat gg 22

<210> 108
<211> 27
<212> DNA
<213> Homo sapiens

<400> 108
ccagttgtga tggataaaat taatcag 27

<210> 109
<211> 27
<212> DNA
<213> Homo sapiens

<400> 109
tgctgtctca ctaccggcca ttctacg 27

<210> 110
<211> 21
<212> DNA
<213> Homo sapiens

<400> 110
tggaaatctg gcaagcagac t 21

<210> 111
<211> 20
<212> DNA
<213> Homo sapiens

<400> 111
caatcccgtt gcctttgttg 20

<210> 112
<211> 25
<212> DNA
<213> Homo sapiens

<400> 112
tgaacacaca tgctgccgag ctctg 25

<210> 113
<211> 27
<212> DNA
<213> Homo sapiens

<400> 113
cttctaggag aacccaacat tgatagt 27

<210> 114
<211> 27
<212> DNA
<213> Homo sapiens

<400> 114
gtttcttgca ggtacttctt aaaagct 27

<210> 115
<211> 30

<212> DNA
<213> Homo sapiens

<400> 115
tattcacatc ccctcagttg cagtgaattg 30

<210> 116
<211> 27
<212> DNA
<213> Homo sapiens

<400> 116
ctgtggctct taatttattg cataatg 27

<210> 117
<211> 26
<212> DNA
<213> Homo sapiens

<400> 117
ttagtgcttt ttgctttcaa gatctt 26

<210> 118
<211> 23
<212> DNA
<213> Homo sapiens

<400> 118
tctcaccttg accctcagcc aag 23

<210> 119
<211> 26
<212> DNA
<213> Homo sapiens

<400> 119
acatttgaca aatttccctt aggatt 26

<210> 120
<211> 20
<212> DNA
<213> Homo sapiens

<400> 120
ccaggacgca gctttaccaa 20

<210> 121
<211> 783
<212> DNA
<213> Homo sapiens

<400> 121

```
ggcacgagcg agttcctgtc tctctgccaa cgccgcccgg atggcttccc aaaaccgcga      60
cccagccgcc actagcgtcg ccgccgcccg taaaggagct gagccgagcg ggggcgccgc     120
ccggggtccg gtgggcaaaa ggctacagca ggagctgatg accctcatga tgtctggcga     180
taaagggatt tctgccttcc ctgaatcaga caacctttc aaatgggtag ggaccatcca      240
tggagcagct ggaacagtat atgaagacct gaggtataag ctctcgctag agttccccag     300
tggctaccct tacaatgcgc ccacagtgaa gttcctcacg ccctgctatc accccaacgt     360
ggacacccag ggtaacatat gcctggacat cctgaaggaa aagtggtctg ccctgtatga     420
tgtcaggacc attctgctct ccatccagag ccttctagga gaacccaaca ttgatagtcc     480
cttgaacaca catgctgccg agctctggaa aaaccccaca gcttttaaga agtacctgca     540
agaaacctac tcaaagcagg tcaccagcca ggagccctga cccaggctgc ccagcctgtc     600
cttgtgtcgt ctttttaatt tttccttaga tggtctgtcc tttttgtgat ttctgtatag     660
gactctttat cttgagctgt ggtatttttg ttttgttttt gtcttttaaa ttaagcctcg     720
gttgagccct tgtatattaa ataaatgcat tttgtcctt ttttaaaaaa aaaaaaaaaa     780
aaa                                                                   783
```

<210> 122
<211> 14796
<212> DNA
<213> Homo sapiens

<400> 122

tctagacatg cggatatatt caagctgggc acagcacagc agccccaccc caggcagctt 60
gaaatcagag ctggggtcca aagggaccac accccgaggg actgtgtggg ggtcggggca 120
cacaggccac tgcttccccc cgtctttctc agccattcct gaagtcagcc tcactctgct 180
tctcagggat ttcaaatgtg cagagactct ggcacttttg tagaagcccc ttctggtcct 240
aacttacacc tggatgctgt ggggctgcag ctgctgctcg ggctcgggag gatgctgggg 300
gcccggtgcc catgagcttt tgaagctcct ggaactcggt tttgagggtg ttcaggtcca 360
ggtggacacc tgggctgtcc ttgtccatgc atttgatgac attgtgtgca gaagtgaaaa 420
ggagttaggc cgggcatgct ggcttatgcc tgtaatccca gcactttggg aggctgaggc 480
gggtggatca cgaggtcagg agttcaatac cagcctggcc aagatggtga aaccccgtct 540
ctactaaaaa tacaaaaaaa ttagccgggc atggtggcgg gcgcatgtaa tcccagctac 600
tggggggggct gaggcagaga attgctggaa cccaggagat ggaggttgca gtgagccaag 660
attgtgccac tgcactgcac tccagcctgg cgacagagca agactctgtc tcaaaaaaaa 720
aaaaaaaaag tgaaaaggag ttgttccttt cctccctcct gagggcaggc aactgctgcg 780
gttgccagtg gaggtggtgc gtccttggtc tgtgcctggg ggccacccca gcagaggcca 840
tggtggtgcc agggcccggt tagcgagcca atcagcagga cccaggggcg acctgccaaa 900
gtcaactgga tttgataact gcagcgaagt taagtttcct gattttgatg attgtgttgt 960
ggttgtgtaa gagaatgaag tatttcgggg tagtatggta atgccttcaa cttacaaacg 1020
gttcaggtaa accacccata tacatacata tacatgcatg tgatatatac acatacaggg 1080
atgtgtgtgt gttcacatat atgaggggag agagactagg ggagagaaag taggttgggg 1140
agagggagag agaaaggaaa acaggagaca gagagagagc ggggagtaga gagagggaag 1200
gggtaagaga gggagaggag gagagaaagg gaggaagaag cagagagtga atgttaaagg 1260
aaacaggcaa aacataaaca gaaaatctgg gtgaagggta tatgagtatt ctttgtacta 1320
ttcttgcaat tatcttttat ttaaattgac atcgggccgg gcgcagtggc tcacatctgt 1380
aatcccagca ctttgggagg ccgaggcagg cagatcactt gaggtcagga gtttgagacc 1440
agcctggcaa acatggtgaa accccatctc tactaaaaat acaaaaatta gcctggtgtg 1500
gtggtgcatg cctttaatct cagctactcg ggaggctgag gcaggagaat cgcttgaacc 1560
cgtggcgggg aggaggttgc agtgagctga gatcatgcca ctgcactcca gcctgggcga 1620
tagagcgaga ctcagtttca aataaataaa taaacatcaa aataaaaagt tactgtatta 1680
aagaatgggg gcggggtggg aggggtgggg agaggttgca aaaataaata aataaataaa 1740

taaaccccaa aatgaaaaag acagtggagg caccaggcct gcgtggggct ggagggctaa 1800
taaggccagg cctcttatct ctggccatag aaccagagaa gtgagtggat gtgatgccca 1860
gctccagaag tgactccaga acaccctgtt ccaaagcaga ggacacactg attttttttt 1920
taataggctg caggacttac tgttggtggg acgccctgct ttgcgaaggg aaaggaggag 1980
tttgccctga gcacaggccc ccaccctcca ctgggctttc cccagctccc ttgtcttctt 2040
atcacggtag tggcccagtc cctggcccct gactccagaa ggtggccctc ctggaaaccc 2100
aggtcgtgca gtcaacgatg tactcgccgg gacagcgatg tctgctgcac tccatccctc 2160
ccctgttcat ttgtccttca tgcccgtctg gagtagatgc tttttgcaga ggtggcaccc 2220
tgtaaagctc tcctgtctga cttttttttt tttttagac tgagttttgc tcttgttgcc 2280
taggctggag tgcaatggca caatctcagc tcactgcacc ctctgcctcc cgggttcaag 2340
cgattctcct gcctcagcct cccgagtagt tgggattaca ggcatgcacc accacgccca 2400
gctaattttt gtatttttag tagagacaag gtttcaccgt gatggccagg ctggtcttga 2460
actccaggac tcaagtgatg ctcctgccta ggcctctcaa agtgttggga ttacaggcgt 2520
gagccactgc acccggcctg cacgcgttct ttgaaagcag tcgagggggc gctaggtgtg 2580
ggcagggacg agctggcgcg gcgtcgctgg gtgcaccgcg accacgggca gagccacgcg 2640
gcgggaggac tacaactccc ggcacacccc gcgccgcccc gcctctactc ccagaaggcc 2700
gcggggggtg gaccgcctaa gagggcgtgc gctcccgaca tgccccgcgg cgcgccatta 2760
accgccagat ttgaatcgcg ggacccgttg gcagaggtgg cggcggcggc atgggtgccc 2820
cgacgttgcc ccctgcctgg cagccctttc tcaaggacca ccgcatctct acattcaaga 2880
actggccctt cttggagggc tgcgcctgca ccccggagcg ggtgagactg cccggcctcc 2940
tggggtcccc cacgcccgcc ttgccctgtc cctagcgagg ccactgtgac tgggcctcgg 3000
gggtacaagc cgccctcccc tccccgtcct gtccccagcg aggccactgt ggctgggccc 3060
cttgggtcca ggccggcctc ccctccctgc tttgtcccca tcgaggcctt tgtggctggg 3120
cctcggggtt ccgggctgcc acgtccactc acgagctgtg ctgtcccttg cagatggccg 3180
aggctggctt catccactgc cccactgaga acgagccaga cttggcccag tgtttcttct 3240
gcttcaagga gctggaaggc tgggagccag atgacgaccc catgtaagtc ttctctggcc 3300
agcctcgatg ggctttgttt tgaactgagt tgtcaaaaga tttgagttgc aaagacactt 3360
agtatgggag ggttgctttc caccctcatt gcttcttaaa cagctgttgt gaacggatac 3420
ctctctatat gctggtgcct tggtgatgct tacaacctaa ttaaatctca tttgaccaaa 3480
atgccttggg gtggacgtaa gatgcctgat gcctttcatg ttcaacagaa tacatcagca 3540
gaccctgttg ttgtgaactc ccaggaatgt ccaagtgctt tttttgagat tttttaaaaa 3600

acagtttaat tgaaatataa cctacacagc acaaaaatta ccctttgaaa gtgtgcactt 3660
cacactttcg gaggctgagg cgggcggatc acctgaggtc aggagttcaa gacctgcctg 3720
gccaacttgg cgaaaccccg tctctactaa aaatacaaaa attagccggg catggtagcg 3780
cacgcccgta atcccagcta ctcgggaggc taaggcagga gaatcgcttg aacctgggag 3840
gcggaggttg cagtgagccg agattgtgcc aatgcactcc agcctcggcg acagagcgag 3900
actccgtcat aaaaataaaa aattgaaaaa aaaaaaagaa agaaagcata tacttcagtg 3960
ttgttctgga tttttttctt caagatgcct agttaatgac aatgaaattc tgtactcgga 4020
tggtatctgt ctttccacac tgtaatgcca tattcttttc tcaccttttt ttctgtcgga 4080
ttcagttgct tccacagctt taattttttt cccctggaga atcaccccag ttgtttttct 4140
ttttggccag aagagagtag ctgttttttt tcttagtatg tttgctatgg tggttatact 4200
gcatccccgt aatcactggg aaaagatcag tggtattctt cttgaaaatg aataagtgtt 4260
atgatatttt cagattagag ttacaactgg ctgtcttttt ggactttgtg tggccatgtt 4320
ttcattgtaa tgcagttctg gtaacggtga tagtcagtta tacagggaga ctcccctagc 4380
agaaaatgag agtgtgagct aggggggtccc ttggggaacc cggggcaata atgcccttct 4440
ctgcccttaa tccttacagt gggccgggca cggtggctta cgcctgtaat accagcactt 4500
tgggaggccg aggcgggcgg atcacgaggt caggagatcg agaccatctt ggctaatacg 4560
gtgaaacccc gtctccacta aaaatacaaa aaattagccg ggcgtggtgg tgggcgcctg 4620
tagtcccagc tactcgggag gctgaggcag gagaatggcg tgaacccagg aggcggagct 4680
tgcagtgagc cgagattgca ccactgcact ccagcctggg cgacagaatg agactccgtc 4740
tcaaaaaaaa aaaaaaaaga aaaaaatctt tacagtggat tacataacaa ttccagtgaa 4800
atgaaattac ttcaaacagt tccttgagaa tgttggaggg atttgacatg taattccttt 4860
ggacatatac catgtaacac ttttccaact aattgctaag gaagtccaga taaaatagat 4920
acattagcca cacagatgtg gggggagatg tccacaggga gagagaaggt gctaagaggt 4980
gccatatggg aatgtggctt gggcaaagca ctgatgccat caacttcaga cttgacgtct 5040
tactcctgag gcagagcagg gtgtgcctgt ggagggcgtg gggaggtggc ccgtggggag 5100
tggactgccg ctttaatccc ttcagctgcc tttccgctgt tgttttgatt tttctagaga 5160
ggaacataaa aagcattcgt ccggttgcgc tttcctttct gtcaagaagc agtttgaaga 5220
attaaccctt ggtgaatttt tgaaactgga cagagaaaga gccaagaaca aaattgtatg 5280
tattgggaat aagaactgct caaaccctgt tcaatgtctt tagcactaaa ctacctagtc 5340
cctcaaaggg actctgtgtt ttcctcagga agcatttttt tttttttct gagatagagt 5400
ttcactcttg ttgcccaggc tggagtgcaa tggtgcaatc ttggctcact gcaacctctg 5460
cctctcgggt tcaagtgatt ctcctgcctc agcctcccaa gtaactggga ttacagggaa 5520

gtgccaccac acccagctaa tttttgtatt tttagtagag atggggtttc accacattgc 5580

ccaggctggt cttgaactcc tgacctcgtg attcgcccac cttggcctcc caaagtgctg 5640

ggattacagg cgtgaaccac cacgcctggc tttttttttt ttgttctgag acacagtttc 5700

actctgttac ccaggctgga gtagggtggc ctgatctcgg atcactgcaa cctccgcctc 5760

ctgggctcaa gtgatttgcc tgcttcagcc tcccaagtag ccgagattac aggcatgtgc 5820

caccacaccc aggtaatttt tgtatttttg gtagagacga ggtttcacca tgttggccag 5880

gctggttttg aactcctgac ctcaggtgat ccacccgcct cagcctccca aagtgctgag 5940

attataggtg tgagccacca cacctggcct caggaagtat ttttattttt aaatttattt 6000

atttatttga gatggagtct tgctctgtcg cccaggctag agtgcagcga cgggatctcg 6060

gctcactgca agctccgccc cccaggttca agccattctc ctgcctcagc ctcccgagta 6120

gctgggacta caggcgcccg ccaccacacc cggctaattt ttttgtattt ttagtagaga 6180

cgggttttca ccgtgttagc caggagggtc ttgatctcct gacctcgtga tctgcctgcc 6240

tcggcctccc aaagtgctgg gattacaggt gtgagccacc acacccggct atttttattt 6300

ttttgagaca gggactcact ctgtcacctg ggctgcagtg cagtggtaca ccatagctca 6360

ctgcagcctc gaactcctga gctcaagtga tcctcccacc tcatcctcac aagtaattgg 6420

gactacaggt gcaccccacc atgcccacct aatttattta tttatttatt tatttatttt 6480

catagagatg agggttccct gtgttgtcca ggctggtctt gaactcctga gctcacggga 6540

tccttttgcc tgggcctccc aaagtgctga gattacaggc atgagccacc gtgcccagct 6600

aggaatcatt tttaaagccc ctaggatgtc tgtgtgattt taaagctcct ggagtgtggc 6660

cggtataagt atataccggt ataagtaaat cccacatttt gtgtcagtat ttactagaaa 6720

cttagtcatt tatctgaagt tgaaatgtaa ctgggcttta tttatttatt tatttattta 6780

tttattttta attttttttt ttgagacgag tctcactttg tcacccaggc tggagtgcag 6840

tggcacgatc tcggctcact gcaacctctg cctcccgggg tcaagcgatt ctcctgcctt 6900

agcctcccga gtagctggga ctacaggcac gcaccaccat gcctggctaa tttttgtatt 6960

tttagtagac ggggtttcac catgctggcc aagctggtct caaactcctg accttgtgat 7020

ctgcccgctt tagcctccca gagtgctggg attacaggca tgagccacca tgcgtggtct 7080

ttttaaaatt ttttgatttt tttttttttt gagacagagc cttgctctgt cgcccaggct 7140

ggagtgcagt ggcacgatct cagctcacta caagctccgc ctcccgggtt cacgccattc 7200

ttctgcctca gcctcctgag tagctgggac tacaggtgcc caccaccacg cctggctaat 7260

tttttttggt atttttatta gagacaaggt ttcatcatgt tggccaggct ggtctcaaac 7320

tcctgacctc aagtgatctg cctgcctcgg cctcccaaag cgctgagatt acaggtgtga 7380

tctactgcgc caggcctggg cgtcatatat tcttatttgc taagtctggc agccccacac 7440
agaataagta ctgggggatt ccatatcctt gtagcaaagc cctgggtgga gagtcaggag 7500
atgttgtagt tctgtctctg ccacttgcag actttgagtt taagccagtc gtgctcatgc 7560
tttccttgct aaatagaggt tagacccccct atcccatggt ttctcaggtt gcttttcagc 7620
ttgaaaattg tattcctttg tagagatcag cgtaaaataa ttctgtcctt atatgtggct 7680
ttattttaat ttgagacaga gtgtcactca gtcgcccagg ctggagtgtg gtggtgcgat 7740
cttggctcac tgcgacctcc acctcccagg ttcaagcgat tctcgtgcct caggctccca 7800
agtagctgag attataggtg tgtgccacca ggcccagcta acttttgtat ttttagtaga 7860
gacagggttt tgccatgttg gctaagctgg tctcgaactc ctggcctcaa gtgatctgcc 7920
cgccttggca tcccaaagtg ctgggattac aggtgtgaac caccacacct ggcctcaata 7980
tagtggcttt taagtgctaa ggactgagat tgtgttttgt caggaagagg ccagttgtgg 8040
gtgaagcatg ctgtgagaga gcttgtcacc tggttgaggt tgtgggagct gcagcgtggg 8100
aactggaaag tgggctgggg atcatctttt tccaggtcag ggtcagccca gcttttctgc 8160
agcgtgccat agaccatctc ttagccctcg tgggtcagag tctctgttgc atattgtctt 8220
ttgttgtttt tcacaacctt ttagaaacat aaaaagcatt cttagcccgt gggctggaca 8280
aaaaaaggcc atgacgggct gtatggattt ggcccagcag gcccttgctt gccaagccct 8340
gttttagaca aggagcagct tgtgtgcctg gaaccatcat gggcacaggg gaggagcaga 8400
gtggatgtgg aggtgtgagc tggaaaccag gtcccagagc gctgagaaag acagagggtt 8460
tttgcccttg caagtagagc aactgaaatc tgacaccatc cagttccaga aagccctgaa 8520
gtgctggtgg acgctgcggg gtgctccgct ctagggttac agggatgaag atgcagtctg 8580
gtagggggag tccactcacc tgttggaaga tgtgattaag aaaagtagac tttcagggcc 8640
gggcatggtg gctcacgcct gtaatcccag cactttggga ggccgaggcg ggtggatcac 8700
gaggtcagga gatcgagacc atcctggcta acatggtgaa accccgtctt tactaaaaat 8760
acaaaaaatt agctgggcgt ggtggcgggc gcctgtagtc ccagctactc gggaggctga 8820
ggcaggagaa tggcgtgaac ctgggaggtg gagcttgctg tgagccgaga tcgcgccact 8880
gcactccagc ctgggcgaca gagcgagact ccgtctcaaa aaaaaaaaaa aaagtaggct 8940
ttcatgatgt gtgagctgaa ggcgcagtag gcagaagtag aggcctcagt ccctgcagga 9000
gacccctcgg tctctatctc ctgatagtca gacccagcca cactggaaag aggggagaca 9060
ttacagcctg cgagaaaagt agggagattt aaaaactgct tggcttttat tttgaactgt 9120
ttttttgtt tgtttgtttt ccccaattca gaatacagaa tacttttatg gatttgtttt 9180
tattacttta attttgaaac aatataatct tttttttgtt gtttttttga gacagggtct 9240
tactctgtca cccaggctga gtgcagtggt gtgatcttgg ctcacctcag cctcgacccc 9300

ctgggctcaa atgattctcc cacctcagct tcccaagtag ctgggaccac aggtgcgtgt 9360

gttgcgctat acaaatcctg aagacaagga tgctgttgct ggtgatgctg gggattccca 9420

agatcccaga tttgatggca ggatgcccct gtctgctgcc ttgccagggt gccaggaggg 9480

cgctgctgtg gaagctgagg cccggccatc cagggcgatg cattgggcgc tgattcttgt 9540

tcctgctgct gcctcggtgc ttagcttttg aaacaatgaa ataaattaga accagtgtga 9600

aaatcgatca gggaataaat ttaatgtgga aataaactga acaacttagt tcttcataag 9660

agtttacttg gtaaatactt gtgatgagga caaaacgaag cactagaagg agaggcgagt 9720

tgtagacctg ggtggcagga gtgttttgtt tgttttcttt ggcagggtct tgctctgttg 9780

ctcaggctgg agtacagtgg cacaatcaca gctcactata gcctcgacct cctggactca 9840

agcaatcctc ctgcctcagc ctcccagtag ctgggactac aggcgcatgc caccatgcct 9900

ggctaatttt aaattttttt ttttctcttt tttgagatgg aatctcactc tgtcgcccag 9960

gctggagtgc agtggcgtga tctcggctga cggcaagctc cgcctcccag gttcactcca 10020

ttcgcctgcc tcagcctccc aagtagctgg gactacaggc gctgggatta caaacccaaa 10080

cccaaagtgc tgggattaca ggcgtgagcc actgcacccg gcctgttttg tctttcaata 10140

gcaagagttg tgtttgcttc gcccctacct ttagtggaaa aatgtataaa atggagatat 10200

tgacctccac attggggtgg ttaaattata gcatgtatgc aaaggagctt cgctaattta 10260

aggctttttt gaaagagaag aaactgaata atccatgtgt gtatatatat tttaaaagcc 10320

atggtcatct ttccatatca gtaaagctga ggctccctgg gactgcagag ttgtccatca 10380

cagtccatta taagtgcgct gctgggccag gtgcagtggc ttgtgcctga atcccagcac 10440

tttgggaggc caaggcagga ggattcattg agcccaggag ttttgaggcg agcctgggca 10500

atgtggccag acctcatctc ttcaaaaaat acacaaaaaa ttagccaggc atggtggcac 10560

gtgcctgtag tctcagctac tcaggaggct gaggtgggag gatcactttg agccttgcag 10620

gtcaaagctg cagtaagcca tgatcttgcc actgcattcc agcctggatg acagagcgag 10680

accctgtctc taaaaaaaaa aaaaaccaaa cggtgcactg ttttcttttt tcttatcaat 10740

ttattatttt taaattaaat tttcttttaa taatttataa attataaatt tatattaaaa 10800

aatgacaaat ttttattact tatacatgag gtaaaactta ggatatataa agtacatatt 10860

gaaaagtaat tttttggctg gcacagtggc tcacacctgt aatcccagca ctttgggagg 10920

ccgtggcggg cagatcacat gagatcatga gttcgagacc aacctgacca acatggagag 10980

accccatctc tactaaaaat acaaaattag ccggggtggt ggcgcatgcc tgtaatccca 11040

gctactcggg aggctgaggc aggagaatct cttgaacccg ggaggcagag gttgcggtga 11100

gccaagatcg tgcctttgca caccagccta ggcaacaaga gcgaaagtcc gtctcaaaaa 11160

aaaagtaatt ttttttaagt taacctctgt cagcaaacaa atttaaccca ataaaggtct 11220

ttgtttttta atgtagtaga ggagttaggg tttataaaaa atatggtagg gaagggggtc 11280

cctggatttg ctaatgtgat tgtcatttgc cccttaggag agagctctgt tagcagaatg 11340

aaaaaattgg aagccagatt cagggaggga ctggaagcaa aagaatttct gttcgaggaa 11400

gagcctgatg tttgccaggg tctgtttaac tggacatgaa gaggaaggct ctggactttc 11460

ctccaggagt ttcaggagaa aggtagggca gtggttaaga gcagagctct gcctagacta 11520

gctggggtgc ctagactagc tggggtgccc agactagctg gggtgcctag actagctggg 11580

tactttgagt ggctccttca gcctggacct cggtttcctc acctgtatag tagagatatg 11640

ggagcaccca gcgcaggatc actgtgaaca taaatcagtt aatggaggaa gcaggtagag 11700

tggtgctggg tgcataccaa gcactccgtc agtgtttcct gttattcgat gattaggagg 11760

cagcttaaac tagagggagt tgagctgaat caggatgttt gtcccaggta gctgggaatc 11820

tgcctagccc agtgcccagt ttatttaggt gctctctcag tgttccctga ttgtttttc 11880

ctttgtcatc ttatctacag gatgtgactg ggaagctctg gtttcagtgt catgtgtcta 11940

ttctttattt ccaggcaaag gaaaccaaca ataagaagaa agaatttgag gaaactgcga 12000

agaaagtgcg ccgtgccatc gagcagctgg ctgccatgga ttgaggcctc tggccggagc 12060

tgcctggtcc cagagtggct gcaccacttc cagggtttat tccctggtgc caccagcctt 12120

cctgtgggcc ccttagcaat gtcttaggaa aggagatcaa cattttcaaa ttagatgttt 12180

caactgtgct cctgttttgt cttgaaagtg gcaccagagg tgcttctgcc tgtgcagcgg 12240

gtgctgctgg taacagtggc tgcttctctc tctctctctc ttttttgggg gctcattttt 12300

gctgttttga ttcccgggct taccaggtga gaagtgaggg aggaagaagg cagtgtccct 12360

tttgctagag ctgacagctt tgttcgcgtg ggcagagcct tccacagtga atgtgtctgg 12420

acctcatgtt gttgaggctg tcacagtcct gagtgtggac ttggcaggtg cctgttgaat 12480

ctgagctgca ggttccttat ctgtcacacc tgtgcctcct cagaggacag tttttttgtt 12540

gttgtgtttt tttgtttttt ttttttggta gatgcatgac ttgtgtgtga tgagagaatg 12600

gagacagagt ccctggctcc tctactgttt aacaacatgg ctttcttatt ttgtttgaat 12660

tgttaattca cagaatagca caaactacaa ttaaaactaa gcacaaagcc attctaagtc 12720

attggggaaa cggggtgaac ttcaggtgga tgaggagaca gaatagagtg ataggaagcg 12780

tctggcagat actccttttg ccactgctgt gtgattagac aggcccagtg agccgcgggg 12840

cacatgctgg ccgctcctcc ctcagaaaaa ggcagtggcc taaatccttt ttaaatgact 12900

tggctcgatg ctgtggggga ctggctgggc tgctgcaggc cgtgtgtctg tcagcccaac 12960

cttcacatct gtcacgttct ccacacgggg gagagacgca gtccgcccag gtccccgctt 13020

tctttggagg cagcagctcc cgcagggctg aagtctggcg taagatgatg gatttgattc 13080

gccctcctcc ctgtcataga gctgcagggt ggattgttac agcttcgctg gaaacctctg 13140
gaggtcatct cggctgttcc tgagaaataa aaagcctgtc atttcaaaca ctgctgtgga 13200
ccctactggg tttttaaaat attgtcagtt tttcatcgtc gtccctagcc tgccaacagc 13260
catctgccca gacagccgca gtgaggatga gcgtcctggc agagacgcag ttgtctctgg 13320
gcgcttgcca gagccacgaa ccccagacct gtttgtatca tccgggctcc ttccgggcag 13380
aaacaactga aaatgcactt cagacccact tatttatgcc acatctgagt cggcctgaga 13440
tagacttttc cctctaaact gggagaatat cacagtggtt tttgttagca gaaaatgcac 13500
tccagcctct gtactcatct aagctgctta tttttgatat ttgtgtcagt ctgtaaatgg 13560
atacttcact ttaataactg ttgcttagta attggctttg tagagaagct ggaaaaaaat 13620
ggttttgtct tcaactcctt tgcatgccag gcggtgatgt ggatctcggc ttctgtgagc 13680
ctgtgctgtg ggcagggctg agctggagcc gcccctctca gcccgcctgc cacggccttt 13740
ccttaaaggc catccttaaa accagaccct catggctgcc agcacctgaa agcttcctcg 13800
acatctgtta ataaagccgt aggcccttgt ctaagcgcaa ccgcctagac tttctttcag 13860
atacatgtcc acatgtccat ttttcaggtt ctctaagttg gagtggagtc tgggaagggt 13920
tgtgaatgag gcttctgggc tatgggtgag gttccaatgg caggttagag cccctcgggc 13980
caactgccat cctggaaagt agagacagca gtgcccgctg cccagaagag accagcaagc 14040
caaactggag ccccccattgc aggctgtcgc catgtggaaa gagtaactca caattgccaa 14100
taaagtctca tgtggtttta tctacttttt ttttcttttt cttttttttt gagacaaggc 14160
cttgccctcc caggctggag tgcagtggaa tgaccacagc tcaccgcaac ctcaaattct 14220
tgcgttcaag tgaacctccc actttagcct cccaagtagc tgggactaca ggcgcacgcc 14280
atcacacccg gctaattgaa aaattttttt ttttgtttag atggaatctc actttgttgc 14340
ccaggctggt ctcaaactcc tgggctcaag tgatcatcct gcttcagcgt ccgacttgtt 14400
ggtattatag gcgtgagcca ctgggcctga cctagctacc atttttttaat gcagaaatga 14460
agacttgtag aaatgaaata acttgtccag gatagtcgaa taagtaactt ttagagctgg 14520
gatttgaacc caggcaatct ggctccagag ctgggccctc actgctgaag gacactgtca 14580
gcttgggagg gtggctatgg tcggctgtct gattctaggg agtgagggct gtctttaaag 14640
caccccattc cattttcaga cagctttgtc agaaaggctg tcatatggag ctgacacctg 14700
cctccccaag gcttccatag atcctctctg tacattgtaa ccttttattt tgaaatgaaa 14760
attcacagga agttgtaagg ctagtacagg ggatcc 14796

<210> 123
<211> 2597
<212> DNA

<213> Homo sapiens

<400> 123

gagcagcgcg cgcaagcagg ccaggggaag gtgggcgcag gtgaggggcc gaggtgtgcg 60

caggacttta gccggttgag aaggatcaag caggcatttg gagcacaggt gtctagaaac 120

ttttaagggg ccggttcaag aaggaaaagt tcccttctgc tgtgaaacta tttggcaaga 180

ggctggaggg cccaatggct gcaaaattgc aacccaacat tcccaaagcc aagagtctag 240

atggcgtcac caatgacaga accgcatctc aagggcagtg gggccgtgcc tgggaggtgg 300

actggttttc actggcgagc gtcatcttcc tactgctgtt cgccccccttc atcgtctact 360

acttcatcat ggcttgtgac cagtacagct gcgccctgac cggccctgtg gtggacatcg 420

tcaccggaca tgctcggctc tcggacatct gggccaagac tccacctata acgaggaaag 480

ccgcccagct ctataccttg tgggtcacct tccaggtgct tctgtacacg tctctccctg 540

acttctgcca taagtttcta cccggctacg taggaggcat ccaggagggg gccgtgactc 600

ctgcaggggt tgtgaacaag tatcagatca acggcctgca agcctggctc ctcacgcacc 660

tgctctggtt tgcaaacgct catctcctgt cctggttctc gcccaccatc atcttcgaca 720

actggatccc actgctgtgg tgcgccaaca tccttggcta tgccgtctcc accttcgcca 780

tggtcaaggg ctacttcttc cccaccagcg ccagagactg caaattcaca ggcaatttct 840

tttacaacta catgatgggc atcgagttta accctcggat cgggaagtgg tttgacttca 900

agctgttctt caatgggcgc cccgggatcg tcgcctggac cctcatcaac ctgtccttcg 960

cagcgaagca gcgggagctc cacagccatg tgaccaatgc catggtcctg gtcaacgtcc 1020

tgcaggccat ctacgtgatt gacttcttct ggaacgaaac ctggtacctg aagaccattg 1080

acatctgcca tgaccacttc gggtggtacc tgggctgggg cgactgtgtc tggctgcctt 1140

atctttacac gctgcagggt ctgtacttgg tgtaccaccc cgtgcagctg tccaccccgc 1200

acgccgtggg cgtcctgctg ctgggcctgg tgggctacta catcttccgg gtggccaacc 1260

accagaagga cctgttccgc cgcacggatg ggcgctgcct catctggggc aggaagccca 1320

aggtcatcga gtgctcctac acatccgccg acgggcagag gcaccacagc aagctgctgg 1380

tgtcgggctt ctggggcgtg gcccgccact tcaactacgt cggcgacctg atgggcagcc 1440

tggcctactg cctggcctgt ggcggtggcc acctgctgcc ctacttctac atcatctaca 1500

tggccatcct gctgacccac cgctgcctcc gggacgagca ccgctgcgcc agcaagtacg 1560

gccgggactg ggagcgctac accgccgcag tgccttaccg cctgctgcct ggaatcttct 1620

aagggcacgc cctagggaga agccctgtgg ggctgtcaag agcgtgttct gccaggtcca 1680

tgggggctgg catcccagct ccaactcgag gagcctcagt ttcctcatct gtaaactgga 1740

gagagcccag cacttggcag gtgtccagta cctaatcacg ctctgttcct tgcttttgcc 1800

```
ttcaagggaa ttccgagtgt ccagcactgc cgtattgcca gcacagacgg attttctcta    1860
atcagtgtcc ctgggcagga ggatgaccca gtcaccttta ctagtccttt ggagacaatt    1920
tacctgtatt aggagcccag gccacgctac actctgccca cactggtgag caggaggtct    1980
tcccacgccc tgtcattagg ctgcatttac tcttgctaaa taaaagtggg agtggggcgt    2040
gcgcgttatc catgtattgc ctttcagctc tagatccccc tcccctgcct gctctgcagt    2100
cgtgggtggg gcccgtgcgc cgtttctcct tggtagcgtg cacggtgttg aactgggaca    2160
ctggggagaa aggggctttc atgtcgtttc cttcctgctc ctgctgcaca gctgccagga    2220
gtgctctgcc tggagtctgc agacctcaga gaggtcccag cactggctgt ggctttcagg    2280
tgtaggcagg tgggctctgc ttcccgattc cctgtgagcg cccaccctct cgaaagaatt    2340
ttctgtcttg ccctgtgact gtgcagactc tggctcgagc aacccgggga acttcaccct    2400
caggggcctc tccacacctt ctccagcgag gaggtctcag tcccagcctc gggagggcac    2460
ctccttttct gtgctttctt ccctgaggca ttcttcctca tccctagggt gttgtgtaga    2520
actcttttta aactctatgc tccgagtaga gttcatcttt atattaaact tcccctgttc    2580
aaaaaaaaaa aaaaaaa                                                   2597
```

<210> 124
<211> 1778
<212> DNA
<213> Homo sapiens

<400> 124

gaggagggaa aaggcgagca aaaaggaaga gtgggaggag gaggggagca caaaggatcc 60
aggtctcccg acgggaggtt aataccaaga accatgtgtg ccgagcggct gggccagttc 120
atgaccctgg ctttggtgtt ggccaccttt gacccggcgc gggggaccga cgccaccaac 180
ccacccgagg gtccccaaga caggagctcc cagcagaaag gccgcctgtc cctgcagaat 240
acagcggaga tccagcactg tttggtcaac gctggcgatg tggggtgtgg cgtgtttgaa 300
tgtttcgaga acaactcttg tgagattcgg ggcttacatg ggatttgcat gacttttctg 360
cacaacgctg gaaaatttga tgcccagggc aagtcattca tcaaagacgc cttgaaatgt 420
aaggcccacg ctctgcggca caggttcggc tgcataagcc ggaagtgccc ggccatcagg 480
gaaatggtgt cccagttgca gcgggaatgc tacctcaagc acgacctgtg cgcggctgcc 540
caggagaaca cccgggtgat agtggagatg atccatttca aggacttgct gctgcacgaa 600
ccctacgtgg acctcgtgaa cttgctgctg acctgtgggg aggaggtgaa ggaggccatc 660
acccacagcg tgcaggttca gtgtgagcag aactggggaa gcctgtgctc catcttgagc 720
ttctgcacct cggccatcca gaagcctccc acggcgcccc ccgagcgcca gccccaggtg 780
gacagaacca agctctccag ggcccaccac ggggaagcag gacatcacct cccagagccc 840

agcagtaggg agactggccg aggtgccaag ggtgagcgag gtagcaagag ccacccaaac 900
gcccatgccc gaggcagagt cgggggcctt ggggctcagg gaccttccgg aagcagcgag 960
tgggaagacg aacagtctga gtattctgat atccggaggt gaaatgaaag gcctggccac 1020
gaaatctttc ctccacgccg tccattttct tatctatgga cattccaaaa catttaccat 1080
tagagagggg ggatgtcaca cgcaggattc tgtggggact gtggacttca tcgaggtgtg 1140
tgttcgcgga acggacaggt gagatgagac ccctgggccc gtggggtctc aggggtgcct 1200
ggtgaattct gcacttacac gtactcaagg gagcgcgccc gcgttatcct cgtacctttg 1260
tcttctttcc atctgtgaag tcagtgggtg tcggccgctc tgttgtgggg gaggtgaacc 1320
agggaggggc agggcaaggc agggccccca gagctgggcc acacagtggg tgctgggcct 1380
cgccccgaag cttctggtgc agcagcctct ggtgctgtct ccgcggaagt cagggcggct 1440
ggattccagg acaggagtga atgtaaaaat aaatatcgct tagaatgcag gagaagggtg 1500
gagaggaggc aggggccgag gggtgcttg gtgccaaact gaaattcagt ttcttgtgtg 1560
gggcttgcgg ttcagagctc ttggcgaggg tggagggagg agtgtcattt ctatgtgtaa 1620
tttctgagcc attgtactgt ctgggctggg gggacactgt ccaagggagt ggcccctatg 1680
agtttatatt ttaaccactg cttcaaatct cgatttcact ttttttattt atccagttat 1740
atctacatat ctgtcatcta aataaatggc tttcaaac 1778

<210> 125
<211> 3246
<212> DNA
<213> Homo sapiens

<400> 125

```
cgcaccatac cggcgcgggc acctggggag aaatggatgg agaagggacc tggctggaaa      60
gctttgcccc gctgctctgc tccgcccata agaggacccc tgaaatgtcc cgtgcagttt     120
gttcaagtcc cctgtgtgat gaaatgtgcc tctcgcctta cccgtgtgag aatacctgtg     180
gtgtggcagc gagtattttg gtatttgacc tgtccaaaga cgacttgata cctctataat     240
gtaacagaaa aggtcagaaa atattaagca agtagaagtg tggagcatat taagcaagat     300
gaacatctcg ggaagcagct gtggaagccc taactctgca gatacatcta gtgactttaa     360
ggacctttgg acaaaactaa aagaatgtca tgatagagaa gtacaaggtt tacaagtaaa     420
agtaaccaag ctaaaacagg aacgaatctt agatgcacaa agactagaag aattcttcac     480
caaaaatcaa cagctgaggg aacagcagaa agtccttcat gaaaccatta aagttttaga     540
agatcggtta agagcaggct tatgtgatcg ctgtgcagta actgaagaac atatgcggaa     600
aaaacagcaa gagtttgaaa atatccggca gcagaatctt aaacttatta cagaacttat     660
gaatgaaagg aatactctac aggaagaaaa taaaaagctt tctgaacaac tccagcagaa     720
```

aattgagaat gatcaacagc atcaagcagc tgagcttgaa tgtgaggaag acgttattcc 780
agattcaccg ataacagcct tctcattttc tggcgttaac cggctacgaa gaaaggagaa 840
cccccatgtc cgatacatag aacaaacaca tactaaattg gagcactctg tgtgtgcaaa 900
tgaaatgaga aaagtttcca agtcttcaac tcatccacaa cataatccta atgaaaatga 960
aattctagta gctgacactt atgaccaaag tcaatctcca atggccaaag cacatggaac 1020
aagcagctat acccctgata agtcatcttt taatttagct acagttgttg ctgaaacact 1080
tggacttggt gttcaagaag aatctgaaac tcaaggtccc atgagccccc ttggtgatga 1140
gctctaccac tgtctggaag gaaatcacaa gaaacagcct tttgaggaat ctacaagaaa 1200
tactgaagat agtttaagat tttcagattc tacttcaaag actcctcctc aagaagaatt 1260
acctactcga gtgtcatctc ctgtatttgg agctacctct agtatcaaaa gtggtttaga 1320
tttgaataca agtttgtccc cttctctttt acagcctggg aaaaaaaaac atctgaaaac 1380
actccctttt agcaacactt gtatatctag attagaaaaa actagatcaa aatctgaaga 1440
tagtgccctt ttcacacatc acagtcttgg gtctgaagtg aacaagatca ttatccagtc 1500
atctaataaa cagatactta taaataaaaa tataagtgaa tccctaggtg aacagaatag 1560
gactgagtac ggtaaagatt ctaacactga taaacatttg gagcccctga aatcattggg 1620
aggccgaaca tccaaaagga agaaaactga ggaagaaagt gaacatgaag taagctgccc 1680
ccaagcttct tttgataaag aaaatgcttt cccttttcca atggataatc agttttccat 1740
gaatggagac tgtgtgatgg ataaacctct ggatctgtct gatcgatttt cagctattca 1800
gcgtcaagag aaaagccaag gaagtgagac ttctaaaaac aaatttaggc aagtgactct 1860
ttatgaggct ttgaagacca ttccaaaggg cttttcctca agccgtaagg cctcagatgg 1920
caactgcacg ttgcccaaag attccccagg ggagccctgt tcacaggaat gcatcatcct 1980
tcagcccttg aataaatgct ctccagacaa taaaccatca ttacaaataa aagaagaaaa 2040
tgctgtcttt aaaattcctc tacgtccacg tgaaagtttg gagactgaga atgttttaga 2100
tgacataaag agtgctggtt ctcatgagcc aataaaaata caaaccaggt cagaccatgg 2160
aggatgtgaa cttgcatcag ttcttcagtt aaatccatgt agaactggta aaataaagtc 2220
tctacaaaac aaccaagatg tatcctttga aaatatccag tggagtatag atccgggagc 2280
agacctttct cagtataaaa tggatgttac tgtaatagat acaaaggatg gcagtcagtc 2340
aaaattagga ggagagacag tggacatgga ctgtacattg gttagtgaaa ccgttctctt 2400
aaaaatgaag aagcaagagc agaagggaga aaaaagttca aatgaagaaa gaaaaatgaa 2460
tgatagcttg gaagatatgt ttgatcggac aacacatgaa gagtatgaat cctgtttggc 2520
agacagtttc tcccaagcag cagatgaaga ggaggaattg tctactgcca caaagaaact 2580
acacactcat ggtgataaac aagacaaagt caagcagaaa gcgtttgtgg agccgtattt 2640

taaaggtgat gaaagagaga ctagcttgca aaattttcct catattgagg tggttcggaa 2700

aaaagaggag agaagaaaac tgcttgggca cacgtgtaag gaatgtgaaa tttattatgc 2760

agatatgcca gcagaagaaa gagaaaagaa attggcttcc tgctcaagac accgattccg 2820

ctacattcca cccaacacac cagagaattt ttgggaagtt ggttttcctt ccactcagac 2880

ttgtatggaa agaggttata ttaaggaaga tcttgatcct tgtcctcgtc caaaaagacg 2940

tcagccttac aacgcaatat tttctccaaa aggcaaggag cagaagacat agacgttgaa 3000

acagaaacag aaggatgaag gacagttttt tccttcttag ttatttatag ttaaagttgg 3060

tactaaacat tgattttttt gatcttctgt aaatggattt ataaatcagt tttctattga 3120

aaatgtttgt gatattttgc ttttgcacct ttaaaacaat aaggcgcttt cattttgcac 3180

tctaacttaa gagtttttac tttatgtagt gatacctaat acaattttga aaatacaaaa 3240

aaaaaa 3246

<210> 126
<211> 3085
<212> DNA
<213> Homo sapiens

<400> 126

```
gagcagccaa aaggcccgcg gagtcgcgct gggccgcccc ggcgcagctg aaccgggggc     60
cgcgcctgcc aggccgacgg gtctggccca gcctggcgcc aaggggttcg tgcgctgtgg    120
agacgcggag ggtcgaggcg gcgcggcctg agtgaaaccc aatggaaaaa gcatgacatt    180
tagaagtaga agacttagct tcaaatccct actccttcac ttactaattt tgtgatttgg    240
aaatatccgc gcaagatgtt gacgttgcag acttgggtag tgcaagcctt gtttattttc    300
ctcaccactg aatctacagg tgaacttcta gatccatgtg gttatatcag tcctgaatct    360
ccagttgtac aacttcattc taatttcact gcagtttgtg tgctaaagga aaaatgtatg    420
gattattttc atgtaaatgc taattacatt gtctggaaaa caaaccattt tactattcct    480
aaggagcaat atactatcat aaacagaaca gcatccagtg tcacctttac agatatagct    540
tcattaaata ttcagctcac ttgcaacatt cttacattcg gacagcttga acagaatgtt    600
tatggaatca caataatttc aggcttgcct ccagaaaaac ctaaaaattt gagttgcatt    660
gtgaacgagg ggaagaaaat gaggtgtgag tgggatggtg gaagggaaac acacttggag    720
acaaacttca ctttaaaatc tgaatgggca acacacaagt ttgctgattg caaagcaaaa    780
cgtgacaccc ccacctcatg cactgttgat tattctactg tgtattttgt caacattgaa    840
gtctgggtag aagcagagaa tgcccttggg aaggttacat cagatcatat caattttgat    900
cctgtatata aagtgaagcc caatccgcca cataatttat cagtgatcaa ctcagaggaa    960
ctgtctagta tcttaaaatt gacatggacc aacccaagta ttaagagtgt tataatacta   1020
```

aaatataaca ttcaatatag gaccaaagat gcctcaactt ggagccagat tcctcctgaa 1080
gacacagcat ccacccgatc ttcattcact gtccaagacc ttaaaccttt tacagaatat 1140
gtgtttagga ttcgctgtat gaaggaagat ggtaagggat actggagtga ctggagtgaa 1200
gaagcaagtg ggatcaccta tgaagataga ccatctaaag caccaagttt ctggtataaa 1260
atagatccat cccatactca aggctacaga actgtacaac tcgtgtggaa gacattgcct 1320
ccttttgaag ccaatggaaa aatcttggat tatgaagtga ctctcacaag atggaaatca 1380
catttacaaa attacacagt taatgccaca aaactgacag taaatctcac aaatgatcgc 1440
tatctagcaa ccctaacagt aagaaatctt gttggcaaat cagatgcagc tgttttaact 1500
atccctgcct gtgactttca agctactcac cctgtaatgg atcttaaagc attccccaaa 1560
gataacatgc tttgggtgga atggactact ccaagggaat ctgtaaagaa atatatactt 1620
gagtggtgtg tgttatcaga taaagcaccc tgtatcacag actggcaaca agaagatggt 1680
accgtgcatc gcacctattt aagagggaac ttagcagaga gcaaatgcta tttgataaca 1740
gttactccag tatatgctga tggaccagga agccctgaat ccataaaggc ataccttaaa 1800
caagctccac cttccaaagg acctactgtt cggacaaaaa aagtagggaa aaacgaagct 1860
gtcttagagt gggaccaact tcctgttgat gttcagaatg gatttatcag aaattatact 1920
atattttata gaaccatcat tggaaatgaa actgctgtga atgtggattc ttcccacaca 1980
gaatatacat tgtcctcttt gactagtgac acattgtaca tggtacgaat ggcagcatac 2040
acagatgaag gtgggaagga tggtccagaa ttcactttta ctaccccaaa gtttgctcaa 2100
ggagaaattg aagccatagt cgtgcctgtt tgcttagcat tcctattgac aactcttctg 2160
ggagtgctgt tctgctttaa taagcgagac ctaattaaaa aacacatctg gcctaatgtt 2220
ccagatcctt caaagagtca tattgcccag tggtcacctc acactcctcc aaggcacaat 2280
tttaattcaa aagatcaaat gtattcagat ggcaatttca ctgatgtaag tgttgtggaa 2340
atagaagcaa atgacaaaaa gccttttcca gaagatctga aatcattgga cctgttcaaa 2400
aaggaaaaaa ttaatactga aggacacagc agtggtattg gggggtcttc atgcatgtca 2460
tcttctaggc caagcatttc tagcagtgat gaaaatgaat cttcacaaaa cacttcgagc 2520
actgtccagt attctaccgt ggtacacagt ggctacagac accaagttcc gtcagtccaa 2580
gtcttctcaa gatccgagtc tacccagccc ttgttagatt cagaggagcg gccagaagat 2640
ctacaattag tagatcatgt agatggcggt gatggtattt tgcccaggca acagtacttc 2700
aaacagaact gcagtcagca tgaatccagt ccagatattt cacattttga aaggtcaaag 2760
caagtttcat cagtcaatga ggaagatttt gttagactta aacagcagat ttcagatcat 2820
atttcacaat cctgtggatc tgggcaaatg aaaatgtttc aggaagtttc tgcagcagat 2880

gcttttggtc caggtactga gggacaagta gaaagatttg aaacagttgg catggaggct 2940
gcgactgatg aaggcatgcc taaaagttac ttaccacaga ctgtacggca aggcggctac 3000
atgcctcagt gaaggactag tagttcctgc tacaacttca gcagtaccta taaagtaaag 3060
ctaaaatgat tttatctgtg aattc 3085

<210> 127
<211> 585
<212> DNA
<213> Homo sapiens

<400> 127

ctgagactga cctgcaggac gaaaccatga agagcctgat ccttcttgcc atcctggccg 60
ccttagcggt agtaactttg tgttatgaat cacatgaaag catggaatct tatgaactta 120
atcccttcat taacaggaga aatgcaaata ccttcatatc ccctcagcag agatggagag 180
ctaaagtcca agagaggatc cgagaacgct ctaagcctgt ccacgagctc aatagggaag 240
cctgtgatga ctacagactt tgcgaacgct acgccatggt ttatggatac aatgctgcct 300
ataatcgcta cttcaggaag cgccgaggga ccaaatgaga ctgagggaag aaaaaaaatc 360
tctttttttc tggaggctgg cacctgattt tgtatccccc tgtagcagca ttactgaaat 420
acataggctt atatacaatg cttctttcct gtatattctc ttgtctggct gcacccctttt 480
ttcccgcccc cagattgata agtaatgaaa gtgcactgca gtgagggtca aaggagagtc 540
aacatatgtg attgttccat aataaacttc tggtgtgata ctttc 585

<210> 128
<211> 1208
<212> DNA
<213> Homo sapiens

<400> 128

```
aagcagacac aatggtaaga atggtgcctg tcctgctgtc tctgctgctg cttctgggtc      60
ctgctgtccc ccaggagaac caagatggtc gttactctct gacctatgtc tacactgggc     120
tgtccaagca tgttgaagac gtccccgcgt ttcaggccct tggctcactc aatgacctcc     180
agttctttag atacaacagt aaagacagga agtctcagcc catgggactc tggagacagg     240
tggaaggaat ggaggattgg aagcaggaca gccaacttca gaaggccagg gaggacatct     300
ttatggagac cctgaaagac atcgtggagt attacaacga cagtaacggg tctcacgtat     360
tgcagggaag gtttggttgt gagatcgaga ataacagaag cagcggagca ttctggaaat     420
attactatga tggaaaggac tacattgaat tcaacaaaga aatcccagcc tgggtcccct     480
tcgacccagc agcccagata accaagcaga agtgggaggc agaaccagtc tacgtgcagc     540
gggccaaggc ttacctggag gaggagtgcc ctgcgactct gcggaaatac ctgaaataca     600
gcaaaaatat cctggaccgg caagatcctc cctctgtggt ggtcaccagc caccaggccc     660
caggagaaaa gaagaaactg aagtgcctgg cctacgactt ctacccaggg aaaattgatg     720
tgcactggac tcgggccggc gaggtgcagg agcctgagtt acggggagat gttcttcaca     780
atggaaatgg cacttaccag tcctgggtgg tggtggcagt gcccccgcag gacacagccc     840
cctactcctg ccacgtgcag cacagcagcc tggcccagcc cctcgtggtg ccctgggagg     900
ccagctagga agcaagggtt ggaggcaatg tgggatctca gacccagtag ctgcccttcc     960
tgcctgatgt gggagctgaa ccacagaaat cacagtcaat ggatccacaa ggcctgagga    1020
gcagtgtggg gggacagaca ggaggtggat ttggagaccg aagactggga tgcctgtctt    1080
gagtagactt ggacccaaaa aatcatctca ccttgagccc acccccaccc cattgtctaa    1140
tctgtagaag ctaataaata atcatccctc cttgcctagc aaaaaaaaaa aaaaaaaaaa    1200
aaaaaaaa                                                             1208
```

<210> 129
<211> 3315
<212> DNA
<213> Homo sapiens

<400> 129

```
aataaatttc tctgtgattg gttggtgaag gttttcaaac cggagctgtg ggcgcggcgc      60
tgctctgccg ttgggtgagg cgcggagcga agtgaagggt ggcccaggtg gggccaggct     120
gactgaatgt atctcctagc tatggactaa ataatacatg gggggaaata aacaagtatt     180
catgagggtg aaaatgtgac ccagcaggaa aattacaact attttcaatt gacgttgaat     240
aggatgagtc atggaattta agtgatttac tgaagattat actactggta gatagaagag     300
ctaaagaaag atggatacta tgatgctgaa tgtgcggaat ctgtttgagc agcttgtgcg     360
ccgggtggag attctcagtg aaggaaatga agtccaattt atccagttgg cgaaggactt     420
tgaggatttc cgtaaaaagt ggcagaggac tgaccatgag ctggggaaat acaaggatct     480
tttgatgaaa gcagagactg agcgaagtgc tctggatgtt aagctgaagc atgcacgtaa     540
tcaggtggat gtagagatca aacggagaca gagagctgag gctgactgcg aaaagctgga     600
acgacagatt cagctgattc gagagatgct catgtgtgac acatctggca gcattcaact     660
aagcgaggag caaaaatcag ctctggcttt tctcaacaga ggccaaccat ccagcagcaa     720
tgctgggaac aaaagactat caaccattga tgaatctggt tccattttat cagatatcag     780
ctttgacaag actgatgaat cactggattg ggactcttct ttggtgaaga ctttcaaact     840
gaagaagaga gaaaagaggc gctctactag ccgacagttt gttgatggtc cccctggacc     900
tgtaaagaaa actcgttcca ttggctctgc agtagaccag gggaatgaat ccatagttgc     960
aaaaactaca gtgactgttc ccaatgatgg cgggcccatc gaagctgtgt ccactattga    1020
gactgtgcca tattggacca ggagccgaag gaaaacaggt actttacaac cttggaacag    1080
```

tgactccacc ctgaacagca ggcagctgga gccaagaact gagacagaca gtgtgggcac 1140

gccacagagt aatggaggga tgcgcctgca tgactttgtt tctaagacgg ttattaaacc 1200

tgaatcctgt gttccatgtg gaaagcggat aaaatttggc aaattatctc tgaagtgtcg 1260

agactgtcgt gtggtctctc atccagaatg tcgggaccgc tgtccccttc cctgcattcc 1320

taccctgata ggaacacctg tcaagattgg agagggaatg ctggcagact ttgtgtccca 1380

gacttctcca atgatcccct ccattgttgt gcattgtgta aatgagattg agcaaagagg 1440

tctgactgag acaggcctgt ataggatctc tggctgtgac cgcacagtaa aagagctgaa 1500

agagaaattc ctcagagtga aaactgtacc cctcctcagc aaagtggatg atatccatgc 1560

tatctgtagc cttctaaaag actttcttcg aaacctcaaa gaacctcttc tgacctttcg 1620

ccttaacaga gcctttatgg aagcagcaga aatcacagat gaagacaaca gcatagctgc 1680

catgtaccaa gctgttggtg aactgcccca ggccaacagg gacacattag ctttcctcat 1740

gattcacttg cagagagtgg ctcagagtcc acatactaaa atggatgttg ccaatctggc 1800

taaagtcttt ggccctacaa tagtggccca tgctgtgccc aatccagacc cagtgacaat 1860

gttacaggac atcaagcgtc aacccaaggt ggttgagcgc ctgctttcct tgcctctgga 1920

gtattggagt cagttcatga tggtggagca agagaacatt gacccctac atgtcattga 1980

aaactcaaat gccttttcaa caccacagac accagatatt aaagtgagtt tactgggacc 2040

tgtgaccact cctgaacatc agcttctcaa gactccttca tctagttccc tgtcacagag 2100

agtccgttcc accctcacca agaacactcc tagatttggg agcaaaagca agtctgccac 2160

taacctagga cgacaaggca actttttgc ttctccaatg ctcaagtgaa gtcacatctg 2220

cctgttactt cccagcattg actgactata agaaaggaca catctgtact ctgctctgca 2280

gcctcctgta ctcattacta cttttagcat tctccaggct tttactcaag tttaattgtg 2340

catgagggtt ttattaaaac tatatatatc tccccttcct tctcctcaag tcacataata 2400

tcagcacttt gtgctggtca ttgttgggag cttttagatg agacatcttt ccaggggtag 2460

aagggttagt atggaattgg ttgtgattct ttttggggaa gggggttatt gttcctttgg 2520

cttaaagcca aatgctgctc atagaatgat ctttctctag tttcatttag aactgatttc 2580

cgtgagacaa tgacagaaac cctacctatc tgataagatt agcttgtctc agggtgggaa 2640

gtgggagggc agggcaaaga aaggattaga ccagaggatt taggatgcct ccttctaaga 2700

accagaagtt ctcattcccc attatgaact gagctataat atggagcttt cataaaaatg 2760

ggatgcattg aggacagaac tagtgatggg agtatgcgta gctttgattt ggatgattag 2820

gtctttaata gtgttgagtg gcacaacctt gtaaatgtga aagtacaact cgtatttatc 2880

tctgatgtgc tgctggctga actttgggtt catttggggt caaagccagt tttctttta 2940

aaattgaatt cattctgatg cttggccccc atacccccaa ccttgtccag tggagcccaa 3000

cttctaaagg tcaatatatc atcctttggc atcccaacta acaataaaga gtaggctata 3060

agggaagatt gtcaatattt tgtggtaaga aaagctacag tcatttttc tttgcacttt 3120

ggatgctgaa atttttccca tggaacatag ccacatctag atagatgtga gctttttctt 3180

ctgttaaaat tattcttaat gtctgtaaaa acgattttct tctgtagaat gtttgacttc 3240

gtattgaccc ttatctgtaa aacacctatt tgggataata tttggaaaaa aagtaaatag 3300

ctttttcaaa atgaa 3315

<210> 130
<211> 2128
<212> DNA
<213> Homo sapiens

<400> 130

```
cttggaagac ttgggtcctt gggtcgcagg ctggagtgca atggtgtgat ctcagctcac     60
tgcaacctct gcttcctggg tttaagtgat tctcctgcct cagcctcccg agtagctggg    120
attacaggca tcatggaccg atctaaagaa aactgcattt caggacctgt taaggctaca    180
gctccagttg gaggtccaaa acgtgttctc gtgactcagc aatttccttg tcagaatcca    240
ttacctgtaa atagtggcca ggctcagcgg gtcttgtgtc cttcaaattc ttcccagcgc    300
gttcctttgc aagcacaaaa gcttgtctcc agtcacaagc cggttcagaa tcagaagcag    360
aagcaattgc aggcaaccag tgtacctcat cctgtctcca ggccactgaa taacacccaa    420
aagagcaagc agcccctgcc atcggcacct gaaaataatc ctgaggagga actggcatca    480
aaacagaaaa atgaagaatc aaaaaagagg cagtgggctt tggaagactt tgaaattggt    540
cgccctctgg gtaaaggaaa gtttggtaat gtttatttgg caagagaaaa gcaaagcaag    600
tttattctgg ctcttaaagt gttatttaaa gctcagctgg agaaagccgg agtggagcat    660
cagctcagaa gagaagtaga aatacagtcc caccttcggc atcctaatat tcttagactg    720
tatggttatt tccatgatgc taccagagtc tacctaattc tggaatatgc accacttgga    780
acagtttata gagaacttca gaaactttca aagtttgatg agcagagaac tgctacttat    840
ataacagaat tggcaaatgc cctgtcttac tgtcattcga agagagttat tcatagagac    900
attaagccag agaacttact tcttggatca gctggagagc ttaaaattgc agattttggg    960
tggtcagtac atgctccatc ttccaggagg accactctct gtggcaccct ggactacctg   1020
ccccctgaaa tgattgaagg tcggatgcat gatgagaagg tggatctctg gagccttgga   1080
gttctttgct atgaattttt agttgggaag cctccttttg aggcaaacac ataccaagag   1140
acctacaaaa gaatatcacg ggttgaattc acattccctg actttgtaac agagggagcc   1200
agggacctca tttcaagact gttgaagcat aatcccagcc agaggccaat gctcagagaa   1260
```

gtacttgaac acccctggat cacagcaaat tcatcaaaac catcaaattg ccaaaacaaa 1320

gaatcagcta gcaaacagtc ttaggaatcg tgcaggggga gaaatccttg agccagggct 1380

gccatataac ctgacaggaa catgctactg aagtttattt taccattgac tgctgccctc 1440

aatctagaac gctacacaag aaatatttgt tttactcagc aggtgtgcct taacctccct 1500

attcagaaag ctccacatca ataaacatga cactctgaag tgaaagtagc cacgagaatt 1560

gtgctactta tactggttca taatctggag gcaaggttcg actgcagccg ccccgtcagc 1620

ctgtgctagg catggtgtct tcacaggagg caaatccaga gcctggctgt ggggaaagtg 1680

accactctgc cctgaccccg atcagttaag gagctgtgca ataaccttcc tagtacctga 1740

gtgagtgtgt aacttattgg gttggcgaag cctggtaaag ctgttggaat gagtatgtga 1800

ttctttttaa gtatgaaaat aaagatatat gtacagactt gtatttttc tctggtggca 1860

ttcctttagg aatgctgtgt gtctgtccgg caccccggta ggcctgattg ggtttctagt 1920

cctccttaac cacttatctc ccatatgaga gtgtgaaaaa taggaacacg tgctctacct 1980

ccatttaggg atttgcttgg gatacagaag aggccatgtg tctcagagct gttaagggct 2040

tattttttta aaacattgga gtcatagcat gtgtgtaaac tttaaatatg caaataaata 2100

agtatctatg tcaaaaaaaa aaaaaaa 2128

<210> 131
<211> 586
<212> DNA
<213> Homo sapiens

<400> 131

ccagcctttc agtgcaggct ccagccctcc acccccaccc gagttgcagg atgtcgatga 60

cagacttgct gaacgctgag gacatcaaga aggcggtggg agcctttagc gctaccgact 120

ccttcgacca caaaaagttc ttccaaatgg tcggcctgaa gaaaaagagt gcggatgatg 180

tgaagaaggt gtttcacatg ctggacaagg acaaaagtgg cttcatcgag gaggatgagc 240

tgggattcat cctaaaaggc ttctccccag atgccagaga cctgtctgct aaagaaacca 300

agatgctgat ggctgctgga gacaaagatg gggacggcaa aattggggtt gacgaattct 360

ccactctggt ggctgaaagc taagaagcac tgactgcccc tggtcttcca cctctctgcc 420

ctgaacaccc aatctcggcc cctctcgcca ccctcctgca tttctgttca gttcgtttat 480

gttatttttt actcccccat cccctgtggc cctctaatga caccattctt ctggaaaatg 540

ctggagaagc aataaaggtt gtaccagtca gaaaaaaaaa aaaaaa 586

<210> 132
<211> 817
<212> DNA

<213> Homo sapiens

<400> 132

```
agtcctgcgt ccgggccccg aggcgcagca gggcaccagg tggagcacca gctacgcgtg     60
gcgcagcgca gcgtccctag caccgagcct cccgcagccg ccgagatgct gcgaacagag    120
agctgccgcc ccaggtcgcc cgccggacag gtggccgcgg cgtccccgct cctgctgctg    180
ctgctgctgc tcgcctggtg cgcgggcgcc tgccgaggtg ctccaatatt acctcaagga    240
ttacagcctg aacaacagct acagttgtgg aatgagatag atgatacttg ttcgtctttt    300
ctgtccattg attctcagcc tcaggcatcc aacgcactgg aggagctttg ctttatgatt    360
atgggaatgc taccaaagcc tcaggaacaa gatgaaaaag ataatactaa aaggttctta    420
tttcattatt cgaagacaca gaagttgggc aagtcaaatg ttgtgtcgtc agttgtgcat    480
ccgttgctgc agctcgttcc tcacctgcat gagagaagaa tgaagagatt cagagtggac    540
gaagaattcc aaagtccctt tgcaagtcaa agtcgaggat attttttatt caggccacgg    600
aatggaagaa ggtcagcagg gttcatttaa aatggatgcc agctaatttt ccacagagca    660
atgctatgga atacaaaatg tactgacatt ttgttttctt ctgaaaaaaa tccttgctaa    720
atgtactctg ttgaaaatcc ctgtgttgtc aatgttctca gttgtaacaa tgttgtaaat    780
gttcaatttg ttgaaaatta aaaaatctaa aaataaa                             817
```

<210> 133
<211> 1967
<212> DNA
<213> Homo sapiens

<400> 133

```
gaaggcgtgg ctccctcccg ggccagtgag cctggcgccg ccgcggccgc gtcccagcag      60
cggagtaggg cggcggctgc gccccgcacc atgggggcag cccagcccca gccgcggtaa     120
acgccgacct ccgccgccgc ccgcgcccgt ctgccccctc ccgctgcggc tctctggacg     180
ccatcccctc ctcacctcga agccaacatg aaggagaccc ggggctacgg aggggatgcc     240
cccttctgca cccgcctcaa ccactcctac acaggcatgt gggcgcccga gcgttccgcc     300
gaggcgcggg gcaacctcac gcgccctcca gggtctggcg aggattgcgg atcggtgtcc     360
gtggccttcc cgatcaccat gctgctcact ggtttcgtgg gcaacgcact ggccatgctg     420
ctcgtgtcgc gcagctaccg gcgccgggag agcaagcgca agaagtcctt cctgctgtgc     480
atcggctggc tggcgctcac cgacctggtc gggcagcttc tcaccacccc ggtcgtcatc     540
gtcgtgtacc tgtccaagca gcgttgggag cacatcgacc cgtcggggcg gctctgcacc     600
tttttcgggc tgaccatgac tgttttcggg ctctcctcgt tgttcatcgc cagcgccatg     660
gccgtcgagc gggcgctggc catcagggcg ccgcactggt atgcgagcca catgaagacg     720
cgtgccaccc gcgctgtgct gctcggcgtg tggctggccg tgctcgcctt cgccctgctg     780
ccggtgctgg gcgtgggcca gtacaccgtc cagtggcccg ggacgtggtg cttcatcagc     840
```

```
accgggcgag ggggcaacgg gactagctct tcgcataact ggggcaacct tttcttcgcc     900
tctgcctttg ccttcctggg gctcttggcg ctgacagtca ccttttcctg caacctggcc     960
accattaagg ccctggtgtc ccgctgccgg gccaaggcca cggcatctca gtccagtgcc    1020
cagtggggcc gcatcacgac cgagacggcc attcagctta tggggatcat gtgcgtgctg    1080
tcggtctgct ggtctccgct cctgataatg atgttgaaaa tgatcttcaa tcagacatca    1140
gttgagcact gcaagacaca cacggagaag cagaaagaat gcaacttctt cttaatagct    1200
gttcgcctgg cttcactgaa ccagatcttg gatccttggg tttacctgct gttaagaaag    1260
atccttcttc gaaagttttg ccagatgaga aaaagaagac tcagagagca agctcctctt    1320
cttcccaccc ctactgtgat tgatccttca aggttctgtg ctcagccctt ccgttggttc    1380
ttggatttgt cctttcccgc catgtcttca tcacatccac aacttccact aacacttgcg    1440
agcttcaaac ttcttagaga accctgcagt gtccagctaa gctgatgact tgaagataaa    1500
tctgcctaac cctgggatga agtatctgtg aactattttg acagcagatg aggaattttg    1560
gggaaattaa aacctgcctt tctgccagga tcacatcact ggaagctcca tgactctctt    1620
tttgtaaaag aaaaaaaaat cacagaaaca cccacctccc aaactattct cttttacttc    1680
ttcccccaag cccaccccca aatataactg ttatccagaa gctgttatgt cctgtttcca    1740
tacatgtttt tgtactttta ctatatctac atacatcaat taaacttatg tcctattgtt    1800
ttgtgaattt atatttgcgt atacattatc atatgtaaaa tttgcatttt tttattgaaa    1860
attatgtttc ttgagattta tccacattga aacatggagc tctaaatcgt taattttaac    1920
cgctatagag tattccataa tttgaataaa gcataatttg tttgtac                  1967
```

<210> 134
<211> 3524
<212> DNA
<213> Homo sapiens

<400> 134

```
tctccgtcag ccgcattgcc cgctcggcgt ccggcccccg acccgtgctc gtccgcccgc     60
ccgcccgccc gcccgcgcca tgaacgccaa ggtcgtggtc gtgctggtcc tcgtgctgac    120
cgcgctctgc ctcagcgacg ggaagcccgt cagcctgagc tacagatgcc catgccgatt    180
cttcgaaagc catgttgcca gagccaacgt caagcatctc aaaattctca acactccaaa    240
ctgtgccctt cagattgtag cccggctgaa gaacaacaac agacaagtgt gcattgaccc    300
gaagctaaag tggattcagg agtacctgga gaaagcttta aacaagaggt tcaagatgtg    360
agagggtcag acgcctgagg aacccttaca gtaggagccc agctctgaaa ccagtgttag    420
ggaagggcct gccacagcct cccctgccag ggcagggccc caggcattgc caagggcttt    480
gttttgcaca ctttgccata ttttcaccat ttgattatgt agcaaaatac atgacattta    540
```

```
tttttcattt agtttgatta ttcagtgtca ctggcgacac gtagcagctt agactaaggc     600
cattattgta cttgccttat tagagtgtct ttccacggag ccactcctct gactcagggc     660
tcctgggttt tgtattctct gagctgtgca ggtggggaga ctgggctgag ggagcctggc     720
cccatggtca gccctagggt ggagagccac caagagggac gcctgggggt gccaggacca     780
gtcaacctgg gcaaagccta gtgaaggctt ctctctgtgg gatgggatgg tggagggcca     840
catgggaggc tcacccctt ctccatccac atgggagccg ggtctgcctc ttctgggagg     900
gcagcagggc taccctgagc tgaggcagca gtgtgaggcc agggcagagt gagacccagc     960
cctcatcccg agcacctcca catcctccac gttctgctca tcattctctg tctcatccat    1020
catcatgtgt gtccacgact gtctccatgg ccccgcaaaa ggactctcag gaccaaagct    1080
ttcatgtaaa ctgtgcacca agcaggaaat gaaaatgtct tgtgttacct gaaaacactg    1140
tgcacatctg tgtcttgtgt ggaatattgt ccattgtcca atcctatgtt tttgttcaaa    1200
gccagcgtcc tcctctgtga ccaatgtctt gatgcatgca ctgttccccc tgtgcagccg    1260
ctgagcgagg agatgctcct tgggcccttt gagtgcagtc ctgatcagag ccgtggtcct    1320
ttggggtgaa ctaccttggt tcccccactg atcacaaaaa catggtgggt ccatgggcag    1380
agcccaaggg aattcggtgt gcaccagggt tgaccccaga ggattgctgc cccatcagtg    1440
ctccctcaca tgtcagtacc ttcaaactag ggccaagccc agcactgctt gaggaaaaca    1500
agcattcaca acttgttttt ggtttttaaa acccagtcca caaaataacc aatcctggac    1560
atgaagattc tttcccaatt cacatctaac ctcatcttct tcaccatttg gcaatgccat    1620
catctcctgc cttcctcctg ggccctctct gctctgcgtg tcacctgtgc ttcgggccct    1680
tcccacagga catttctcta agagaacaat gtgctatgtg aagagtaagt caacctgcct    1740
gacatttgga gtgttcccct cccactgagg gcagtcgata gagctgtatt aagccactta    1800
aaatgttcac ttttgacaaa ggcaagcact tgtgggtttt tgttttgttt ttcattcagt    1860
cttacgaata cttttgccct ttgattaaag actccagtta aaaaaaattt taatgaagaa    1920
agtggaaaac aaggaagtca aagcaaggaa actatgtaac atgtaggaag taggaagtaa    1980
attatagtga tgtaatcttg aattgtaact gttcgtgaat ttaataatct gtagggtaat    2040
tagtaacatg tgttaagtat tttcataagt atttcaaatt ggagcttcat ggcagaaggc    2100
aaacccatca acaaaaattg tcccttaaac aaaaattaaa atcctcaatc cagctatgtt    2160
atattgaaaa aatagagcct gagggatctt tactagttat aaagatacag aactctttca    2220
aaacctttg aaattaacct ctcactatac cagtataatt gagttttcag tggggcagtc    2280
attatccagg taatccaaga tattttaaaa tctgtcacgt agaacttgga tgtacctgcc    2340
cccaatccat gaaccaagac cattgaattc ttggttgagg aaacaaacat gaccctaaat    2400
```

```
cttgactaca gtcaggaaag gaatcatttc tatttctcct ccatgggaga aaatagataa   2460
gagtagaaac tgcagggaaa attatttgca taacaattcc tctactaaca atcagctcct   2520
tcctggagac tgcccagcta aagcaatatg catttaaata cagtcttcca tttgcaaggg   2580
aaaagtctct tgtaatccga atctcttttt gctttcgaac tgctagtcaa gtgcgtccac   2640
gagctgttta ctagggatcc ctcatctgtc cctccgggac ctggtgctgc ctctacctga   2700
cactcccttg ggctccctgt aacctcttca gaggccctcg ctgccagctc tgtatcagga   2760
cccagaggaa ggggccagag gctcgttgac tggctgtgtg ttgggattga gtctgtgcca   2820
cgtgtatgtg ctgtggtgtg tccccctctg tccaggcact gagataccag cgaggaggct   2880
ccagagggca ctctgcttgt tattagagat tacctcctga gaaaaaagct tccgcttgga   2940
gcagaggggc tgaatagcag aaggttgcac ctcccccaac cttagatgtt ctaagtcttt   3000
ccattggatc tcattggacc cttccatggt gtgatcgtct gactggtgtt atcaccgtgg   3060
gctccctgac tgggagttga tcgcctttcc caggtgctac accctttcc agctggatga    3120
gaatttgagt gctctgatcc ctctacagag cttccctgac tcattctgaa ggagccccat   3180
tcctgggaaa tattccctag aaacttccaa atcccctaag cagaccactg ataaaaccat   3240
gtagaaaatt tgttattttg caacctcgct ggactctcag tctctgagca gtgaatgatt   3300
cagtgttaaa tgtgatgaat actgtatttt gtattgtttc aagtgcatct cccagataat   3360
gtgaaaatgg tccaggagaa ggccaattcc tatacgcagc gtgctttaaa aaataaataa   3420
gaaacaactc tttgagaaac aacaatttct actttgaagt cataccaatg aaaaaatgta   3480
tatgcactta taattttcct aataaagttc tgtactcaaa tgta                    3524
```

<210> 135
<211> 1705
<212> DNA
<213> Homo sapiens

<400> 135

```
ggctcactgc atctccggct cctggactca agcgattctc ctgcctcagg ctcccaaggt      60
ggcagcacgc aaagggtgtc cctgtccctc aaggggtcat ggcctccatg ttgctcgccc     120
agcggctggc ctgcagcttc cagcacacgt accgcctgct ggtgcctgga tccagacaca     180
ttagtcaagc tgcagccaaa gtcgacgttg aatttgatta tgatgggcct ctgatgaaga     240
cggaagtccc agggcctaga tctcaggagt taatgaaaca gctgaatata attcagaatg     300
cagaggctgt gcattttttc tgcaattacg aagagagccg aggcaattac ctggttgatg     360
tggacggcaa ccgaatgctg gatctttatt cccagatctc ctctgttccc ataggttaca     420
gcgacccggc cctcgtgaaa ctcatccaac agccacaaaa tgcgagcatg tttgtcaaca     480
gacccgccct cgaaatcctg cctccggaga actttgtgga gaagctccgg cagtccttgc     540
tctcggtggc tcccaaaggg atgtcccagc tcatcaccat ggcctgcggc tcctgctcca     600
atgaaaacgc cttaaagacc atcttcatgt ggtaccggag caaggaaaga gggcagaggg     660
gattctccaa agaggagctg gagacgtgca tgattaacca ggccccctgg tgccccgact     720
acagcatcct ctccttcatg ggttccttcc atgggaggac catgggttgc ttagcgacca     780
cgcactctaa agccattcac aagatcgata tcccttcctt tgactggccc atcgcaccgt     840
tcccacggct gaaataccct ctggaagagt ttgtgaaaga gaaccaacag gaagaggccg     900
gctgtctgga agaggttgag gatctgattg tgaaatatcg aaaaaagaag aagacggtgg     960
ccgggatcat cgtggagccc atccagtccg agggtggaga caaccatgca tccgatgact    1020
tctttcggaa gctgagagac atcgccagga agcactgctg cgccttcttg gtggacgagg    1080
tccagaccgg aggaggctgc acgggcaagt tctgggccca tgagcactgg ggcctggatg    1140
acccagcaga cgtgatgacc ttcagcaaga agatgatgac tgggggcttc ttcctcaagg    1200
aggagttcag gcctaatgct ccctaccgga tcttcaacac gtggctgggg gacccgtcca    1260
agaacctgtt gctggctgag gtcatcaaca tcatcaagcg ggaggacctg ctaaataatg    1320
cagcccatgc cgggaaggcc ctgctcacag gactgctgga cctccaggcc cggtaccccc    1380
agttcatcag cagggtgaga ggacgaggca ccttttgctc cttcgatact cccgatgatt    1440
ccatacggaa taagctcatt ttaattgcca gaaacaaagg tgtggtgttg ggtggctgtg    1500
gtgacaaatc cattcgtttc cgtcccacgc tggtgttcag ggatcaccac gctcacctgt    1560
tcctcaatat tttcagtgac atcttagcag acttcaagta aagaagccat ttccactaca    1620
gtgagaaagc ccggatccca acagttgtca aattgattag tttgcctaat tcatgttttc    1680
acttaaaagt atcagaggtg gaatt                                          1705
```

<210> 136
<211> 2808

<212> DNA
<213> Homo sapiens

<400> 136

```
ggaaagcacc tgtgagcttg gcaagtcagt tcagagctcc agcccgctcc agcccggccc     60
gacccgaccg cacccggcgc ctgcctcgct cgggctcccc ggccagccat gggcccttgg    120
agccgcagcc tctcgggcct gctgctgctg ctgaggtctc ctcttggctc tcaggagcgg    180
agccctcctc cctgtttgac gcgagagcta cacgttcacg gtgccccggc gccacctgag    240
aagaggccgc gtctgggcag agtgaatttt gaagattgca ccggtcgaca aaggacagct    300
attttcctga caccgattcc gaaagtgggc acagatggtg tgattacagt caaaaggcct    360
ctacggtttc ataacccaac agatccattt cttggtctac gctgggactc cacctacaga    420
aagttttcca ccaaagtcac gctgaataca gtggggcacc accaccgccc cccgccccat    480
```

caggcctccg tttctggaat ccaagcagaa ttgctcacat ttcccaactc ctctcctggc 540
ctcagaagac agaagagaga ctgggttatt cctcccatca gctgcccaga aaatgaaaaa 600
ggcccatttc ctaaaaacct ggttcagatc aaatccaaca aagacaaaga aggcaaggtt 660
ttctacagca tcactggcca aggagctgac acacccccctg ttggtgtctt tattattgaa 720
agagaaacag gatggctgaa ggtgacagag cctctggata gagaacgcat tgccacatac 780
actctcttct ctcacgctgt gtcatccaac gggaatgcag ttgaggatcc aatggagatt 840
ttgatcacgg taaccgatca gaatgacaac aagcccgaat tcacccagga ggtctttaag 900
gggtctgtca tggaaggtgc tcttccagga acctctgtga tggaggtcac agccacagac 960
gcggacgatg atgtgaacac ctacaatgcc gccatcgctt acaccatcct cagccaagat 1020
cctgagctcc ctgacaaaaa tatgttcacc attaacagga acacaggagt catcagtgtg 1080
gtcaccactg ggctggaccg agagagtttc cctacgtata ccctggtggt tcaagctgct 1140
gaccttcaag gtgaggggtt aagcacaaca gcaacagctg tgatcacagt cactgacacc 1200
aacgataatc ctccgatctt caatcccacc acgtacaagg gtcaggtgcc tgagaacgag 1260
gctaacgtcg taatcaccac actgaaagtg actgatgctg atgcccccaa taccccagcg 1320
tgggaggctg tatacaccat attgaatgat gatggtggac aatttgtcgt caccacaaat 1380
ccagtgaaca acgatggcat tttgaaaaca gcaaagggct tggattttga ggccaagcag 1440
cagtacattc tacacgtagc agtgacgaat gtggtacctt ttgaggtctc tctcaccacc 1500
tccacagcca ccgtcaccgt ggatgtgctg gatgtgaatg aaggccccat ctttgtgcct 1560
cctgaaaaga gagtggaagt gtccgaggac tttggcgtgg gccaggaaat cacatcctac 1620
actgcccagg agccagacac atttatggaa cagaaaataa catatcggat ttggagagac 1680
actcgcaact ggctggagat taatccggac actggtgcca tttccactcg ggctgagctg 1740
gacagggagg attttgagca cgtgaagaac agcacgtaca cagccctaat catagctaca 1800
gacaatggtt ctccagttgc tactggaaca gggacacttc tgctgatcct gtctgatgtg 1860
aatgacaacg cccccatacc agaacctcga actatattct tctgtgagag gaatccaaag 1920
cctcaggtca taaacattca tgatgcagac cttcctccca atacatctcc cttcacagca 1980
gaactaacac acgggcgagt gcccaactgg accattcagt acaacgaccc aacccaagaa 2040
tctatcattt tgaagccaaa gatggcctta gaggtgggtg actacaaaat caatctcaag 2100
ctcatggata accagaataa agaccaagtg accaccttag aggtcagcgt gtgtgactgt 2160
gaaggggccg ccggcgtctg taggaaggca cagcctgtcg aagcaggatt gcaaattcct 2220
gccattctgg ggattcttgg aggaattctt gctttgctaa ttctgattct gctgctcttg 2280
ctgtttcttc ggaggagagc ggtggtcaaa gagcccttac tgcccccaga ggatgacacc 2340
cgggacaacg tttattacta tgatgaagaa ggaggcggag aagaggacca ggactttgac 2400

```
ttgagccagc tgcacagggg cctggacgct cggcctgaag tgactcgtaa cgacgttgca   2460
ccaaccctca tgagtgtccc ccggtatctt ccccgccctg ccaatcccga tgaaattgga   2520
aattttattg atgaaaatct gaaagcggct gatactgacc ccacagcccc gccttatgat   2580
tctctgctcg tgtttgacta tgaaggaagc ggttccgaag ctgctagtct gagctccctg   2640
aactcctcag agtcagacaa agaccaggac tatgactact tgaacgaatg gggcaatccg   2700
ttcaagaagc tggctgacat gtacggaggc ggcgaggacc actaggggac tcgagagagg   2760
cggcccagac catgtgcaga aatgcagaaa tcagcgttct ggtgtttt                2808
```

<210> 137
<211> 591
<212> DNA
<213> Homo sapiens

<400> 137

```
cttctctggg acacattgcc ttctgttttc tccagcatgc gcttgctcca gctcctgttc     60
agggccagcc ctgccaccct gctcctggtt ctctgcctgc agttgggggc caacaaagct    120
caggacaaca ctcggaagat cataataaag aattttgaca ttcccaagtc agtacgtcca    180
aatgacgaag tcactgcagt gcttgcagtt caaacagaat tgaaagaatg catggtggtt    240
aaaacttacc tcattagcag catccctcta caaggtgcat ttaactataa gtatactgcc    300
tgcctatgtg acgacaatcc aaaaaccttc tactgggact tttacaccaa cagaactgtg    360
caaattgcag ccgtcgttga tgttattcgg gaattaggca tctgccctga tgatgctgct    420
gtaatcccca tcaaaaacaa ccggttttat actattgaaa tcctaaaggt agaataatgg    480
aagccctgtc tgtttgccac acccaggtga tttcctctaa agaaacttgg ctggaatttc    540
tgctgtggtc tataaaataa acttcttaac atgcttaaaa aaaaaaaaaa a             591
```